# EUROPEAN PATENT SPECIFICATION

(11) **EP 2 238 250 B1**
(45) Date of publication and mention of the grant of the patent: **19.07.2017**
(21) Application number: 08866608.6
(22) Date of filing: 22.12.2008
(51) Int. Cl.: C12N 15/113, A61K 38/17, A61P 41/00, A61P 19/02

(54) **USE OF ANTI-CONNEXIN 43 POLYNUCLEOTIDES, PEPTIDES OR ANTIBODIES FOR THE TREATMENT OF ORTHOPEDIC CONDITIONS**
VERWENDUNG VON ANTI-CONNEXIN 43-POLYNUKLEOTIDEN, -PEPTIDEN ODER ANTIKÖRPERN ZUR BEHANDLUNG ORTHOPÄDISCHER LEIDEN
UTILISATION DE POLYNUCLÉOTIDES, PEPTIDES OU ANTICORPS ANTI-CONNEXINE 43TRAITEMENT D'AFFECTIONS ORTHOPÉDIQUES

(30) Priority: 21.12.2007 US 8660 P
(43) Date of publication of application: 13.10.2010
(73) Proprietor: CoDa Therapeutics, Inc., San Diego, CA 92130 (US)
(72) Inventor: BECKER, David, L., Abbots Langley Hertfordshire WD50GG (GB); GREEN, Colin, R., Auckland 1023 (NZ); DUFT, Bradford, J., Rancho Santa Fe CA 92067 (US)
(74) Representative: J A Kemp
(86) International application number: PCT/US2008/014020
(87) International publication number: WO 2009/085269

(56) References cited:
- WO-A-00/44409
- WO-A-2006/134494
- WO-A-2008/060622
- WAGGETT A.D. ET AL.: "Connexin 32 and 43 gap junctions differentially modulate tenocyte esponse to cyclic mechanical load." EUR. J. CELL. BIOL., vol. 085, 2006, pages 1145-1154, XP002516102
- QIU C ET AL: "Targeting Connexin43 Expression Accelerates the Rate of Wound Repair" CURRENT BIOLOGY, CURRENT SCIENCE, GB, vol. 13, no. 19, 30 September 2003 (2003-09-30), pages 1697-1703, XP004545249 ISSN: 0960-9822

## Description

### FIELD

The inventions relate to orthopedic diseases, disorders and conditions and thereof and related pharmaceutical compositions, the use of said compositions in therapy, formulations, articles of manufacturer and kits comprising such compositions.

### BACKGROUND

The following includes information that may be useful in understanding the present invention. It is not an admission that any of the information provided herein is prior art, or relevant, to the presently described or claimed inventions, or that any publication or document that is specifically or implicitly referenced is prior art.

In humans and other mammals wound injury triggers an organized complex cascade of cellular and biochemical events that will in most cases result in a healed wound. An ideally healed wound is one that restores normal anatomical structure, function, and appearance at the cellular, tissue, organ, and organism levels. Wound healing, whether initiated by surgery, trauma, microbes or foreign materials, proceeds via a complex process encompassing a number of overlapping phases, including inflammation, epithelialization, angiogenesis and matrix production and deposition. Normally, these processes lead to a mature wound and a certain degree of scar formation. Although inflammation and repair mostly occur along a prescribed course, the sensitivity of the process is dependent on the balance of a variety of wound healing modulating factors, including for example, a network of regulatory cytokines and growth factors.

Normal function of a joint and its movement can be severely impaired by inflammation, scarring and/or abnormal tissue formation that takes place both inside and/or around the joint (intra-articular or peri-articular) or any other affected area during the subsequent wound healing process following orthopedic surgical procedures. This may result in tenderness, aching, pain, and lengthy recovery times, as well as the loss of joint mobility or reduced range of motion, tonicity, or elasticity of the joint/articular structures, such as for example, muscle, tendon, capsule, bone, or ligament. Reduced joint mobility may also involve permanently altered or shortened joint or tissue architecture.

Altered or abnormal joint mobility or joint architecture may also be associated with or caused by a variety of injuries and conditions such as, for example, metabolic disorders, ischemia, trauma, injury to joint, capsule, bone, cartilage, tendon, ligament, or muscle, fractures, subluxation, dislocation, crush injuries, prolonged immobilization (*e.g.,* immobilization of a joint in a cast or splint), and paralysis.

To date, common surgical interventions to alleviate altered or abnormal joint mobility or joint architecture caused by scarring and abnormal tissue formation have been met with limited success as corrective surgical procedure is also a form of controlled injury or trauma and the procedure often ignites or reignites inflammation and proliferation in the tissue and the reformation of the scar and tissue abnormality around the point of articulation.

In certain cases, reduced joint motion may have hereditary components and the primary scar and/or the abnormal tissue growth may take place around and/or on the outside of the joint. One such condition is the Dupytren's contracture in which the connective tissue in the palmer aspect of the hand begins to scar and thicken and eventually leading to deformation of the hand at the site of the thickening, and the resultant loss of range of motion of the fingers. Treatment for contractures is limited to after a contracture is already established. Surgical release procedures include manipulation under anesthesia which involves putting the patient to sleep and breaking down the adhesion by forcing the joint (forced manipulation). This often reignites the inflammation and proliferation in the tissue and the reformation of the scar and stiffness. Surgical interventions may also include an open surgical procedure that involves releasing (open release) and removing the restricting scar and abnormal tissue (debulking). This operation may also be performed through an arthroscope, where the scar and restricting tissue is released and removed using special tools (athroscopic release). Unfortunately, surgical interventions fail, and may actually make the condition worse as the surgery itself is a controlled injury or trauma, which can cause even more scarring and abnormal tissue formation in response to the surgical injury. Treatments such as physiotherapy and range of motion exercises are used but with limited success. Pharmacological therapy has also been attempted with limited or no success. Agents most often used include non-steriodal anti-inflammatories, steroids and radiation.

Gap junctions are cell membrane structures that facilitate direct cell-cell communication. A gap junction channel is formed of two connexins (hemichannels), each composed of six connexin subunits. Each hexameric connexin docks with a connexin in the opposing membrane to form a single gap junction. Gap junction channels are reported to be found throughout the body. Tissue such as the corneal epithelium, for example, has six to eight cell layers, yet expresses different gap junction channels in different layers with connexin 43 in the basal layer and connexin 26 from the basal to middle wing cell layers. In general, connexins are a family of proteins, commonly named according to their molecular weight or classified on a phylogenetic basis into alpha, beta, and gamma subclasses. At least 20 human and 19 murine isoforms have been identified. Different tissues and cell types are reported to have characteristic patterns of connexin protein expression and tissues such as cornea have been shown to alter connexin protein expression pattern following injury or transplantation (Qui, C. et al., (2003) Current Biology, 13:1967-1703; Brander et al., (2004), J. Invest Dermatol. 122:1310-20).

Antisense technology has been reported for the modulation of the expression for genes implicated in viral, fungal and metabolic diseases. See, *e.g.,* U.S. Pat. No. 5,166,195, (oligonucleotide inhibitors of HIV), U.S. Pat. No. 5,004,810 (oligomers for hybridizing to herpes simplex virus Vmw65 mRNA and inhibiting replication). See also U.S. Pat. No. 7,098,190 to Becker et al. (formulations comprising antisense nucleotides to connexins). Peptide inhibitors (including peptidomimetics) of gap junctions and hemichannels have been reported. See for example Berthoud, V.M. et al., Am J. Physiol. Lung Cell Mol. Physiol. 279: L619 - L622 (2000); Evans, W.H. and Boitano, S. Biochem. Soc. Trans. 29: 606 - 612, and De Vriese A.S., et al. Kidney Int. 61: 177 - 185 (2001). See also Green and Becker, WO2006/134494 ("Anti-connexin compounds and methods of use").

Despite advances in the understanding of the principles of the mechanisms underlying the wound healing processes associated with orthopedic procedures, there remains a significant unmet need for suitable therapeutic options for improving outcomes and recoveries.

### BRIEF SUMMARY

The inventions described and claimed herein have many attributes and embodiments including, but not limited to, those set forth or described or referenced in this Brief Summary. It is not intended to be all-inclusive and the inventions described and claimed herein are not limited to or by the features or embodiments identified in this Brief Summary, which is included for purposes of illustration only and not restriction.

The invention generally relates to compositions for the treatment of a subject suffering from, predisposed to, or at risk for various orthopedic-related diseases, disorders, or conditions. In one aspect, the invention relates to a composition comprising an effective amount of an anti-connexin 43 agent for use in a method of reducing pain and/or improving mobility in a subject having or suspected of having an orthopedic disease, disorder, or condition characterized in whole or in part by pain and/or excess scarring inside and/or around a joint. In the case of established disease, disorders or conditions, the compositions of the invention can be used to complement a release procedure (*e.g.,* forced manipulation, open release, arthroscopic release, or debulking of scar) to prevent the recurrence abnormal tissue formation and/or further contracture formation in and/or around a joint. For example, in one embodiment of the invention, compositions are provided for treating a Carpal Tunnel Syndrome (*e.g.,* Carpal Tunnel release surgery), contracture in and/or around a joint ("joint contracture"), or recurrence. In on embodiment, the composition is administered to the site of the injury before, at the time of or after a release procedure.

In one embodiment, the composition is administered to the site of the injury before, at the time of or after an orthopedic surgery, *e.g.,* a release procedure, an arthroscopic procedure, a joint surgery (*e.g.,* hip, shoulder or knee surgery, including replacement procedures). In general, orthopedic surgeries addressed with the inventions described and claimed herein include hand surgery; shoulder and elbow surgery; total joint reconstruction (arthroplasty); foot and ankle surgery; spine surgery; surgical sports medicine; and orthopedic trauma. Thus, for example, orthopedic surgeries include knee arthroscopy and meniscectomy; shoulder arthroscopy and decompression; carpal tunnel release; knee arthroscopy and chondroplasty; removal of support implants; knee arthroscopy and anterior cruciate ligament reconstruction; knee replacement; repair of femoral neck fractures; repair of trochanteric fractures; debridement of skin/muscle/bone/fracture; knee arthroscopy repair of both menisci; hip replacement; shoulder arthroscopy/distal clavicle excision; repair of rotator cuff tendon; repair fracture of radius/ulna; laminectomy; repair of ankle fracture (bimalleolar type); shoulder arthroscopy and débridement; lumbar spinal fusion; repair fracture of the distal radius; low back intervertebral disc surgery; incise finger tendon sheath; repair of ankle fracture (fibula); repair of femoral shaft fracture; repair of trochanteric fracture. Total hip replacement, total shoulder replacement, and total knee replacement are included as well, as is uni-compartment knee replacement, in which only one side of an arthritic knee is replaced, and joint replacements for other joints, including elbow, wrist, ankle, and fingers. Also included in orthopedic surgeries is bone grafting, a surgical procedure that replaces missing bone with material from the patient's own body, or an artificial, synthetic, or natural substitute.

Repeat applications are included within the invention.

Arthroscopic applications, topical applications, and instillations are also included within the invention.

The invention also relates to compositions and their use promoting healing following an orthopedic surgical procedure. In one aspect the invention relates to a composition comprising an anti-connexin 43 agent for use in a method of improving recovery time, reducing pain, decreasing joint contracture and/or improving mobility in a subject undergoing an orthopedic surgery comprising administration of said composition into or around the surgical site within said subject before, at the time of, or after said orthopedic procedure. In one embodiment, administration of an anti-connexin agent is effective to decrease or prevent, in whole or in part, joint contraction in a post-operative subject. In one embodiment, administration of an anti-connexin agent is effective to improve recovery time in a post-operative subject. In one embodiment, administration of an anti-connexin agent is effective to decrease pain in a post-operative subject. In one embodiment, administration of an anti-connexin agent is effective to improve overall recovery result in a post-operative subject. In one embodiment, improved recovery results comprises increased post-operative mobility.

In one aspect the invention relates to compositions for decreasing post-orthopedic-surgical joint contracture by administering to a subject an anti-connexin agent.

The anti-connexin compositions may decrease post-orthopedic-surgical vascular damage at the surgical site or improve or maintain vascular integrity during and/or following an orthopedic-surgical procedure.

In certain aspects of the invention, compositions herein described may additionally include one or more therapeutic agents. Therapeutic agents may include, for example, anti-infectives, anesthetics, analgesics, antibiotics, narcotics, and steroidal and non-steroidal anti-inflammatory agents. In another aspect of the invention, compositions may also include one or more agents useful for wound healing. Agents useful for wound healing may include, for example, a wound healing associated growth factor, and/or a cytokine.

Methods of treatment described herein may further comprise administration of a second composition. The second composition conveniently comprises one or more therapeutic agents, one or more agents useful for wound healing, and/or one ore more anti-microtubule agents.

The second composition may be administered before, after, and/or simultaneously with the first composition. The second composition may be administered before and after the first composition. The second composition may also be administered simultaneously with the first composition. For example, a composition comprising an anti-connexin agent effective in treating contracture may be combined in its use with a second composition having one or more drugs effective in treating contractors or one or more of the associated conditions.

Anti-connexin agents and compositions may be administered alone or in combination with other therapeutic agents into or around the affected site, including for example, by topical application, instillation, or injection, for example, peritendinal injection, soft tissue injection, intra-articular injection and peri-articular injection. Anti-connexin agents compositions may also be administered alone or in combination with other therapeutic agents using inter-operative techniques. Administration may also occur via injection or inter-operatively into articular capsules or bone recesses. Anti-connexin agents compositions may be administered as mono-therapy, concurrent or intra-operatively in combination with corrective orthopedic procedures, or post-procedurally.

Anti-connexin agents useful in all aspects of the invention as set forth herein include, for example, anti-connexin polynucleotides, as well as antibodies and binding fragments thereof, and peptides and polypeptides, including peptidomimetics and peptide analogs.

Examples of a connexin antisense polynucleotide include an anti-connexin oligodeoxynucleotide (ODN), including antisense (including modified and unmodified backbone antisense; *e.g*., a DNA antisense polynucleotide that binds to a connexin mRNA), RNAi, and siRNA polynucleotides.

Suitable connexin antisense polynucleotides include for example, antisense ODN against connexin 43. Other antisense polynucleotides include antisense against connexin 26, connexin 37, connexin 30, connexin 31.1 and connexin 32. In certain embodiments, suitable compositions include multiple connexin antisense polynucleotides in combination, including for example, polynucleotides targeting Connexin 43, and connexins 26, 37, 30, and 31.1.

Aspects of the invention are described with reference to oligodeoxynucleotides. Oligonucleotides include modified and unmodified backbone oligonucleotides, *e.g*., a DNA antisense polynucleotide that binds to a connexin mRNA. However it is understood that other suitable polynucleotides (such as RNA polynucleotides, *e.g.,* RNAi and siRNA) may be used in these aspects.

Conveniently, the oligodeoxynucleotide to connexin 43 is selected from: GTA ATT GCG GCA AGA AGA ATT GTT TCT GTC (SEQ.ID.NO:1); GTA ATT GCG GCA GGA GGA ATT GTT TCT GTC (SEQ.ID.NO:2); and GGC AAG AGA CAC CAA AGA CAC TAC CAG CAT (SEQ.ID.NO:3).

According to one preferred aspect, the oligodeoxynucleotide to connexin 43 is: GTA ATT GCG GCA AGA AGA ATT GTT TCT GTC (SEQ.ID.NO:1).

In one aspect, the invention provides a formulation for use in therapeutic treatment, which formulation comprises at least one anti-connexin agent together with a pharmaceutically acceptable carrier or vehicle or diluent to provide a pharmaceutical composition. In one preferred form, the formulation contains anti-connexin polynucleotides to one connexin protein only. Most preferably, this connexin protein is connexin 43. Alternatively, the formulation contains oligodeoxynucleotides to more than one connexin protein.

Accordingly, in another aspect, the invention provides a formulation comprising at least one connexin antisense polynucleotide to a connexin protein together with a pharmaceutically acceptable carrier or vehicle.

The invention includes a formulation comprising a pharmaceutically acceptable anti-connexin agent, *e.g.,* a connexin polynucleotide, preferably a connexin antisense polynucleotide, in an amount effective to promote healing, prevent, and/or decrease pain, prevent and/or decrease vascular damage, prevent and/or improve recovery time, and/or improver overall recovery outcome (*e.g.* increase post-operative mobility) and/or prevent and/or decrease abnormal tissue formation in and/or around a joint in a post-orthopedic-surgical subject. Such formulations include, for example, topical delivery forms and formulations, including gel formulations and wash solution formulations. The anti-connexin polynucleotides and connexin antisense polynucleotides are anti-connexin 43 polynucleotides and connexin 43 antisense polynucleotides.

The compositions described herein may be formulated to provide sustained release. In one aspect, the invention includes compositions comprising an effective amount of an anti-connexin agent formulated in a delayed release preparation, a slow release preparation, an extended release preparation, a controlled release preparation, and/or in a repeat action preparation. These may be applied topically or instilled, for example.

Compositions and methods that employ anti-connexin agents in combination with other therapeutic agents, agents useful for wound healing, and/or anti-microtubule agents are disclosed and claimed.

The invention includes pharmaceutical compositions and formulations comprising (a) a therapeutically effective amount of an anti-connexin 43 agent, and (b) a therapeutically effective amount of one or more therapeutic agents. The invention includes pharmaceutical compositions and formulations comprising (a) a therapeutically effective amount of an anti-connexin 43 agent, and (b) a therapeutically effective amount of one or more agents useful for wound healing. The invention includes pharmaceutical compositions and formulations comprising (a) a therapeutically effective amount of an anti-connexin 43 agent, and (b) a therapeutically effective amount of a anti-microtubule agent. Preferably, the pharmaceutical compositions and formulations further comprise a pharmaceutically acceptable carrier, diluent or excipient.

Pharmaceutical compositions and formulations are provided for combined, simultaneous, separate sequential, or sustained administration. In one embodiment, a composition or formulation comprising an anti-connexin agent is administered at or about the same time as one ore more therapeutic agents, agents useful for wound healing, and/or anti-microtubule agents.

Pharmaceutical compositions and formulations are also provided in the form of a combined preparation, for example, as an admixture of an anti-connexin agent and one or more other agents useful for wound healing, *e.g*., growth factors that are effective in promoting or improving wound healing, such as platelet derived growth factor, epidermal growth factor, fibroblast growth factor (*e.g.,* FGF2), vascular endothelial growth factor, and transforming growth factor β3, and/or cytokines that are effective in promoting or improving wound healing, such as IL-7 and IL-10, and/or other agents that are effective in promoting or improving wound healing, such as IGF *(e.g.,* IGF-1) and IGFBP *(e.g.,* IGFBP-2).

The term "a combined preparation" includes a "kit of parts" in the sense that the combination partners as defined above can be dosed independently or by use of different fixed combinations with distinguished amounts of the combination partners (a) and (b), *i. e.* simultaneously, separately or sequentially. The parts of the kit can then, for example, be administered simultaneously or chronologically staggered, that is at different time points and with equal or different time intervals for any part of the kit of parts.

The anti-connexin agent and therapeutic agents, agents useful for wound healing, and/or anti-microtubule agents may be administered to the patient simultaneously, sequentially or separately. If administered separately, preferably the anti-connexin agent and the therapeutic agent, agent useful for wound healing and/or anti-microtubule agent are administered sequentially. Preferably, the agents are administered sequentially within at least about 1 minute, 5 minutes, 15 minutes, one-half hour of each other, or one hour. The agents may also be administered with about two to eight hours of each other, with about one day to about one week of each other, or as otherwise deemed appropriate. Preferably, the anti-connexin agent is administered first. Preferably, the anti-connexin agent is an anti-connexin 43 agent.

The anti-connexin agent may be used in combination with dressings and matrices. In one aspect, the invention comprises a synthetic or naturally occurring wound healing matrix comprising an anti-connexin 43 polynucleotide, for example, an anti-connexin 43 polynucleotide. Preferred anti-connexin polynucleotides include anti-connexin 43 oligodeoxynucleotides.

The anti-connexin agent and one or more therapeutics agents, agents useful for wound healing, and/or anti-microtubule agents may be used in combination with dressings and matrices.

In another aspect, the application describes an article of manufacture comprising a vessel containing a therapeutically effective amount of an anti-connexin agent and instructions for use, including use for the treatment of a subject. In one embodiment, the vessel further comprises one or more therapeutic agents, agents useful for wound healing, and/or microtubule agents. The article of manufacture may comprise a second vessel containing a therapeutically effective amount one or more therapeutic agents, agents useful for wound healing, and/or microtubule agents.

The application describes an article of manufacture comprising packaging material containing one or more dosage forms containing one or more pharmaceutically acceptable anti-connexin agents, wherein the packaging material has a label that indicates that the dosage form can be used for a post-orthopedic-surgical subject. Such dosage forms include, for example, topical delivery and instillation forms and formulations. Said article may further comprise one or more dosage forms containing one or more therapeutic agents, agents useful for wound healing, and/or microtubule agents.

These and other aspects of the present inventions, which are not limited to or by the information in this Brief Summary, are provided below.

### DETAILED DESCRIPTION

Orthopedic diseases, disorders and/or conditions and the procedures performed to treat said diseases, disorders and/or conditions can be debilitating and lead to loss of joint function and range of motion, as well as slow and difficult recovery times. It has been discovered that certain compounds, including those described or referenced herein, can prevent and/or decrease pain, vascular damage, and/or abnormal tissue formation in a subject.

Subjects that may be treated using the compounds, compositions, and methods of treatment described herein include, for example, subjects suffering from, predisposed to or at risk of orthopedic diseases, disorders and/or conditions, and subjects who have undergone or will undergo an orthopedic surgical procedure.

As used herein, "subject" refers to any mammal, including humans, domestic and farm animals, and zoo, sports, or pet animals, such as dogs, horses, cats, sheep, pigs, cows, *etc.* The preferred mammal herein is a human, including adults, children, and the elderly.

As used herein, "preventing" means preventing in whole or in part, or ameliorating or controlling, or reducing or halting the progress of a condition.

As used herein, an "effective amount" or "therapeutically effective amount" in reference to the compounds or compositions of the instant invention refers to the amount sufficient to induce a desired biological, pharmaceutical, or therapeutic result. That result can be alleviation of the signs, symptoms, or causes of a disease or disorder or condition, or any other desired alteration of a biological system. In the present invention, the result will involve the promotion of healing, preventing and/or decreasing pain, preventing and/or decreasing vascular damage, preventing and/or decreasing abnormal tissue formation inside and/or around a joint and/or preventing, decreasing or reversing of joint contracture in whole or in part, and/or improving recovery times, and/or improving overall recovery results, including increased post-operative mobility. The inventions described and claimed herein can also improve recovery times for post-orthopedic-surgical patients.

As used herein, the term "treating" refers to both therapeutic treatment and prophylactic or preventative measures.

As used herein, "simultaneously" is used to mean that the one or more gap junction modulating agents, *e.g*., peptides or blockers, are administered concurrently, whereas the term "in combination" is used to mean they are administered, if not simultaneously or in physical combination, then "sequentially" within a timeframe that they both are available to act therapeutically. Thus, administration "sequentially" may permit one agent to be administered within minutes (for example, 1, 2, 3, 4, 5, 10, 15, 20, 25, 30) minutes or a matter of hours, days, weeks or months after the other provided that one or more gap junction modulating agents are concurrently present in effective amounts. The time delay between administration of the components will vary depending on the exact nature of the components, the interaction there between, and their respective half-lives.

The terms "peptidomimetic and "mimetic" include naturally occurring and synthetic chemical compounds that may have substantially the same structural and functional characteristics of protein regions which they mimic. In the case of connexins, these may mimic, for example, the extracellular loops of opposing connexins involved in connexin-connexin docking and cell-cell channel formation, and/or the extracellular loops of hemichannel connexins.

As used herein, the term "peptide analogs" refer to the compounds with properties analogous to those of the template peptide and can be non-peptide drugs. "Peptidomimetics" (also known as peptide mimetics) which include peptide-based compounds, also include such non-peptide based compounds such as peptide analogs. Peptidomimetics that are structurally similar to therapeutically useful peptides can be used to produce an equivalent or enhanced therepeutic or prophylactic effect. Generally, peptidomimetics are structural or functional mimics (*e.g.* identical or similar) to a paradigm polypeptide (*i.e*, a polypeptide that has a biological or pharmacological function or activity), but can also have one or more peptide linkages optionally replaced by a linkage selected from the group consisting of, for example, -CH₂NH-, CH₂S-, -CH₂-CH₂-, -CH=CH- (cis and trans), -COCH₂-, -CH(OH)CH₂-, and -CH₂SO-. The mimetic can be either entirely composed of natural amino acids, synthetic chemical compounds, non-natural analogues of amino acids, or, is a chimeric molecule of partly natural peptide amino acids and partly non-natural analogs of amino acids. The mimetic can also comprise any amount of natural amino acid conservative substitutions as long as such substitutions also do not substantially alter mimetic activity. In the case of connexins, these can mimic, for example, the extracellular loops of opposing connexins involved in connexin-connexin docking and cell-cell channel formation. For example, a mimetic composition can be useful as a gap junction modulating agent if it is capable of down-regulating biological actions or activities of connexins, such as, for example, preventing the docking of connexins to form gap-junction-mediated cell-cell communications, or preventing the opening of connexins to expose the cell cytoplasm to the extracellular millieu. Peptidomimetics encompass those described herein, as well as those as may be known in the art, whether now known or later developed.

In general, the terms "modulator" and "modulation" of gap junction activity, as used herein in its various forms, refers to inhibition in whole or in part of the action or activity of a gap junction or a hemichannel and may function as gap junction modulating agents.

As used herein, the term "protein" refers to any polymer of two or more individual amino acids (whether or not naturally occurring) linked via peptide bonds, as occur when the carboxyl carbon atom of the carboxylic acid group bonded to the alpha-carbon of one amino acid (or amino acid residue) becomes covalently bound to the amino nitrogen atom of the amino group bonded to the alpha-carbon of an adjacent amino acid. These peptide bond linkages, and the atoms comprising them (*i.e.,* alpha-carbon atoms, carboxyl carbon atoms (and their substituent oxygen atoms), and amino nitrogen atoms (and their substituent hydrogen atoms)) form the "polypeptide backbone" of the protein. In addition, as used herein, the term "protein" is understood to include the terms "polypeptide" and "peptide" (which, at times, may be used interchangeably herein). Similarly, protein fragments, analogs, derivatives, and variants are may be referred to herein as "proteins," and shall be deemed to be a "protein" unless otherwise indicated. The term "fragment" of a protein refers to a polypeptide comprising fewer than all of the amino acid residues of the protein. A "domain" of a protein is also a fragment, and comprises the amino acid residues of the protein often required to confer activity or function.

Orthopedic diseases, disorders, and conditions are well known in the art and may include, for example, anterior cruciate ligament (acl) injury; arthritis of the shoulder; articular cartilage injury of the knee; bowlegs; broken back; broken hip (pelvis); broken leg; broken neck; bunions; bursitis; carpal tunnel syndrome; chronic low back pain; clubfoot; curvature of the spine (scoliosis); diabetic foot; dislocated elbow; dislocated hip; dupuytren's contracture; flatfoot; foot deformity; forearm fractures in children; hemophiliac arthritis; herniated disc (slipped disc); hip labral tear; hip arthritis; infectious arthritis; inflammatory hip conditions; intoeing; knee arthritis; knock knee; meniscal tear; osteoarthritis; osteonecrosis; osteoporosis; rheumatoid arthritis; rotator cuff injuries; scoliosis; shoulder arthritis; shoulder instability; shoulder pain; slipped disc (herniated disc); spinal stenosis/degenerative spondylolisthesis; spondylolisthesis; sports injuries to foot; sprained ankle; tendonitis; thumb arthritis; trigger finger, and joint contracture.

An orthopedic disease, disorder or condition also includes pain, vascular damage, swelling, inflammation, scarring and joint contractures which arise after an orthopedic injury. Representative examples of injuries which may give rise to inflammation, scarring and joint contractures include, for example, trauma (*e.g.,* crushes, cuts, tears, disruptions, impacts, and tractions, especially in or around a joint), a fracture (which may occur in or around a joint, such as an elbow or hip), a subluxation, a dislocation (*e.g.,* in the finger, elbow, shoulder, ankle, knee, or hip), or a joint (*e.g.,* shoulder, elbow, hip, temporomandibular joint, facet, finger, knee, ankle, or toe) disruption and other bone, cartilage, tendon or ligament injuries, or there may be no identifiable cause (*e.g.,* frozen shoulder).

Orthopedic surgical procedures are also well known in the art and include, for example, all procedures and/or therapeutic modalities presently known or later developed for treating a subject suffering from orthopedic related disease, disorders, or conditions, exemplary procedures may include; arthroscopy; artificial disc replacement; autologous chondrocyte implantation; bone graft; bunion removal; carpal tunnel release; clubfoot repair; corticosteroids (injection and medication); functional restoration program; hip arthroscopy; hip replacement; knee arthroscopy; minimally invasive total hip replacement; partial knee replacement; partial hip replacement; shoulder arthroscopy; shoulder joint replacement; total hip replacement; total knee replacement; wrist arthroscopy; knee arthroscopy and meniscectomy; shoulder arthroscopy and decompression; carpal tunnel release; knee arthroscopy and chondroplasty; removal of support implant; knee arthroscopy and anterior cruciate ligament reconstruction; knee replacement; repair of femoral neck fracture; repair of trochanteric fracture; débridement of skin/muscle/bone/fracture; knee arthroscopy repair of both menisci; hip replacement; shoulder arthroscopy/distal clavicle excision; repair of rotator cuff tendon; repair fracture of radius (bone)/ulna; laminectomy; repair of ankle fracture (bimalleolar type); shoulder arthroscopy and débridement; lumbar spinal fusion; repair fracture of the distal part of radius; low back intervertebral disc surgery; incise finger tendon sheath; repair of ankle fracture (fibula); repair of femoral shaft fracture; repair of trochanteric fracture as well as all procedures associated with general, specialized, or modified hand surgery; shoulder and elbow surgery; total joint reconstruction (arthroplasty); pediatric orthopedics; foot and ankle surgery; spine surgery; musculoskeletal oncology; surgical sports medicine; and orthopedic trauma.

### Anti-Connexin Agents

Anti-connexin agents of the invention described herein are capable of modulating or affecting the transport of molecules into and out of cells (*e.g.*, blocking or inhibiting or downregulating). Thus, certain anti-connexin agents described herein modulate cellular communication (*e.g.,* cell to cell). Certain anti-connexin agents are gap junction modulation agents. Certain anti-connexin agents modulate or effect transmission of molecules between the cell cytoplasm and the periplasmic or extracellular space. Such anti-connexin agents are generally targeted to connexins and/or connexin hemichannels (connexons) or to gap junction themselves. Hemichannels and resulting gap junctions that comprise connexins are independently involved in the release or exchange of small molecules between the cell cytoplasm and an extracellular space or tissue in the case of open hemichannels, and between the cytoplasm of adjoining cell in the case of open gap junctions. Thus, an anti-connexin agents provided herein may directly or indirectly reduce coupling and communication between cells or reduce or block communication (or the transmission of molecules) between a cell and extracellular space or tissue, and the modulation of transport of molecules from a cell into an extracellular space or tissue (or from an extracellular space or tissue into a cell) or between adjoining cells is within the scope of anti-connexin agents and embodiments of the invention. In the present invention, the connexin is connexin 43.

Any anti-connexin agent that is capable of eliciting a desired inhibition of the passage (*e.g.* transport) of molecules through a gap junction or connexin hemichannel may be used in embodiments of the invention. Any anti-connexin agents that modulates the passage of molecules through a gap junction or connexin hemichannel are also provided in particular embodiments (*e.g.,* those that modulate, block or lessen the passage of molecules from the cytoplasm of a cell into an extracellular space or adjoining cell cytoplasm). Such anti-connexin agents may modulate the passage of molecules through a gap junction or connexin hemichannel with or without gap junction uncoupling (blocking the transport of molecules through gap junctions). Such compounds include, for example, proteins and polypeptides, polynucleotides, and other organic compounds, and they may, for example block the function or expression of a gap junction or a hemichannel in whole or in part, or downregulate the production of a connexin in whole or in part. Certain gap junction inhibitors are listed in Evans, W.H. and Boitano, S. Biochem. Soc. Trans. 29: 606-612 (2001). Other compounds include connexin phosphorylation compounds that close gap junctions and/or hemichannels, in whole or in part, and connexin carboxy-terminal polypeptides. In the present invention, the connexin is connexin 43.

Certain anti-connexin agents provide downregulation of connexin expression (for example, by downregulation of mRNA transcription or translation) or otherwise decrease or inhibit the activity of a connexin protein, a connexin hemichannel or a gap junction. In the case of downregulation, this will have the effect of reducing direct cell-cell communication by gap junctions, or exposure of cell cytoplasm to the extracellular space by hemichannels, at the site at which connexin expression is downregulated. Anti-connexin 43 agents are preferred.

Examples of anti-connexin agents include agents that decrease or inhibit expression or function of connexin mRNA and/or protein or that decrease activity, expression or formation of a connexin, a connexin hemichannel or a gap junction. Anti-connexin agents include anti-connexin polynucleotides, such as antisense polynucleotides and other polynucleotides (such as polynucleotides having siRNA or ribozyme functionalities), as well as antibodies and binding fragments thereof, and peptides and polypeptides, including peptidomimetics and peptide analogs that modulate hemichannel or gap junction activity or function. Anti-connexin 43 agents are preferred.

### Anti-Connexin Polynucleotides

Anti-connexin polynucleotides include connexin antisense polynucleotides as well as polynucleotides which have functionalities which enable them to downregulate connexin expression. Other suitable anti-connexin polynucleotides include RNAi polynucleotides and siRNA polynucleotides. Anti-connexin 43 polynucleotides are used in the invention.

Synthesis of antisense polynucleotides and other anti-connexin polynucleotides such as RNAi, siRNA, and ribozyme polynucleotides as well as polynucleotides having modified and mixed backbones is known to those of skill in the art. *See e.g.* Stein C.A. and Krieg A.M. (eds), Applied Antisense Oligonucleotide Technology, 1998 (Wiley-Liss). Methods of synthesizing antibodies and binding fragments as well as peptides and polypeptides, including peptidomimetics and peptide analogs are known to those of skill in the art. See *e.g.* Lihu Yang et al., Proc. Natl. Acad. Sci. U.S.A., 1; 95(18): 10836-10841 (Sept 1 1998); Harlow and Lane (1988) "Antibodies: A Laboratory Manuel" Cold Spring Harbor Publications, New York; Harlow and Lane (1999) "Using Antibodies" A Laboratory Manuel, Cold Spring Harbor Publications, New York.

According to one aspect, the downregulation of connexin expression may be based generally upon the antisense approach using antisense polynucleotides (such as DNA or RNA polynucleotides), and more particularly upon the use of antisense oligodeoxynucleotides (ODN). These polynucleotides (*e.g.,* ODN) target the connexin protein (s) to be downregulated. Typically the polynucleotides are single stranded, but may be double stranded.

The antisense polynucleotide may inhibit transcription and/or translation of a connexin. Preferably the polynucleotide is a specific inhibitor of transcription and/or translation from the connexin gene or mRNA, and does not inhibit transcription and/or translation from other genes or mRNAs. The product may bind to the connexin gene or mRNA either (i) 5' to the coding sequence, and/or (ii) to the coding sequence, and/or (iii) 3' to the coding sequence.

The antisense polynucleotide is generally antisense to a connexin 43 mRNA. Such a polynucleotide may be capable of hybridizing to the connexin mRNA and may thus inhibit the expression of connexin by interfering with one or more aspects of connexin mRNA metabolism including transcription, mRNA processing, mRNA transport from the nucleus, translation or mRNA degradation. The antisense polynucleotide typically hybridizes to the connexin mRNA to form a duplex which can cause direct inhibition of translation and/or destabilization of the mRNA. Such a duplex may be susceptible to degradation by nucleases.

The antisense polynucleotide may hybridize to all or part of the connexin mRNA. Typically the antisense polynucleotide hybridizes to the ribosome binding region or the coding region of the connexin mRNA. The polynucleotide may be complementary to all of or a region of the connexin mRNA. For example, the polynucleotide may be the exact complement of all or a part of connexin mRNA. However, absolute complementarity is not required and polynucleotides which have sufficient complementarity to form a duplex having a melting temperature of greater than about 20°C, 30°C or 40°C under physiological conditions are particularly suitable for use in the present invention.

Thus the polynucleotide is typically a homologue of a sequence complementary to the mRNA. The polynucleotide may be a polynucleotide which hybridizes to the connexin mRNA under conditions of medium to high stringency such as 0.03M sodium chloride and 0.03M sodium citrate at from about 50°C to about 60°C.

For certain aspects, suitable polynucleotides are typically from about 6 to 40 nucleotides in length. Preferably a polynucleotide may be from about 12 to about 35 nucleotides in length, or alternatively from about 12 to about 20 nucleotides in length or more preferably from about 18 to about 32 nucleotides in length. According to an alternative aspect, the polynucleotide may be at least about 40, for example at least about 60 or at least about 80, nucleotides in length and up to about 100, about 200, about 300, about 400, about 500, about 1000, about 2000 or about 3000 or more nucleotides in length.

The connexin protein or proteins targeted by the polynucleotide will be dependent upon the site at which downregulation is to be effected. This reflects the nonuniform make-up of gap junction(s) at different sites throughout the body in terms of connexin sub-unit composition. The connexin is a connexin that naturally occurs in a human or animal in one aspect or naturally occurs in the tissue in which connexin expression or activity is to be decreased. The connexin gene (including coding sequence) generally has homology with the coding sequence of one or more of the specific connexins mentioned herein, such as homology with the connexin 43 coding sequence shown in Table 8. The connexin is typically an α or ß connexin. Preferably the connexin is an α connexin and is expressed in the tissue to be treated.

Some connexin proteins are however more ubiquitous than others in terms of distribution in tissue. One of the most widespread is connexin 43. Polynucleotides targeted to connexin 43 are suitable for use in the present invention. In other aspects other connexins are targeted.

Anti-connexin polynucleotides include connexin antisense polynucleotides as well as polynucleotides which have functionalities which enable them to downregulate connexin expression. Other suitable anti-connexin polynucleotides include RNAi polynucleotides and SiRNA polynucleotides.

In one preferred aspect, the antisense polynucleotides are targeted to the mRNA of one connexin protein only. This connexin protein is connexin 43. In another aspect, connexin protein is connexin 26, 30, 31.1, 32, 36, 37, 40, or 45. In other aspects, the connexin protein is connexin 30.3, 31, 40.1, or 46.6.

It is also contemplated that polynucleotides targeted to separate connexin proteins be used in combination (for example 1, 2, 3, 4 or more different connexins may be targeted). For example, polynucleotides targeted to connexin 43, and one or more other members of the connexin family (such as connexin 26, 30, 30.3, 31.1, 32, 36, 37, 40, 40.1, 45, and 46.6) can be used in combination.

Alternatively, the antisense polynucleotides may be part of compositions which may comprise polynucleotides to more than one connexin protein. One of the connexin proteins to which polynucleotides are directed is connexin 43. Other connexin proteins to which oligodeoxynucleotides are directed may include, for example, connexins 26, 30, 30.3, 31.1, 32, 36, 37, 40, 40.1, 45, and 46.6. Suitable exemplary polynucleotides (and ODNs) directed to various connexins are set forth in Table 1.

Individual antisense polynucleotides may be specific to a particular connexin, or may target 1, 2, 3 or more different connexins. Specific polynucleotides will generally target sequences in the connexin gene or mRNA which are not conserved between connexins, whereas non-specific polynucleotides will target conserved sequences for various connexins.

The polynucleotides for use in the invention may suitably be unmodified phosphodiester oligomers. Such oligodeoxynucleotides may vary in length. A 30 mer polynucleotide has been found to be particularly suitable.

Many aspects of the invention are described with reference to oligodeoxynucleotides. However it is understood that other suitable polynucleotides (such as RNA polynucleotides) may be used in these aspects.

The antisense polynucleotides may be chemically modified. This may enhance their resistance to nucleases and may enhance their ability to enter cells. For example, phosphorothioate oligonucleotides may be used. Other deoxynucleotide analogs include methylphosphonates, phosphoramidates, phosphorodithioates, N3'P5'-phosphoramidates and oligoribonucleotide phosphorothioates and their 2'-O-alkyl analogs and 2'-O-methylribonucleotide methylphosphonates. Alternatively mixed backbone oligonucleotides ("MBOs") may be used. MBOs contain segments of phosphothioate oligodeoxynucleotides and appropriately placed segments of modified oligodeoxy-or oligoribonucleotides. MBOs have segments of phosphorothioate linkages and other segments of other modified oligonucleotides, such as methylphosphonate, which is non-ionic, and very resistant to nucleases or 2'-O-alkyloligoribonucleotides. Methods of preparing modified backbone and mixed backbone oligonucleotides are known in the art.

The precise sequence of the antisense polynucleotide used in the invention will depend upon the target connexin protein. In one embodiment, suitable connexin antisense polynucleotides can include polynucleotides such as oligodeoxynucleotides selected from the following sequences set forth in Table 1:

**TABLE 1**

| | | |
|---|---|---|
| 5' GTA ATT GCG GCA AGA AGA ATT GTT TCT GTC 3' | (connexin 43) | (SEQ.ID.NO:1) |
| 5' GTA ATT GCG GCA GGA GGA ATT GTT TCT GTC 3' | (connexin 43) | (SEQ.ID.NO:2) |
| 5' GGC AAG AGA CAC CAA AGA CAC TAC CAG CAT 3' | (connexin 43) | (SEQ.ID.NO:3) |
| 5' TCC TGA GCA ATA CCT AAC GAA CAA ATA 3' | (connexin 26) | (SEQ.ID.NO:4) |
| 5' CAT CTC CTT GGT GCT CAA CC 3' | (connexin 37) | (SEQ.ID.NO:5) |
| 5' CTG AAG TCG ACT TGG CTT GG 3' | (connexin 37) | (SEQ.ID.NO:6) |
| 5' CTC AGA TAG TGG CCA GAA TGC 3' | (connexin 30) | (SEQ.ID.NO:7) |
| 5' TTG TCC AGG TGA CTC CAA GG 3' | (connexin 30) | (SEQ.ID.NO:8) |
| 5' CGT CCG AGC CCA GAA AGA TGA GGT C 3' | (connexin 31.1) | (SEQ.ID.NO:9) |
| 5' AGA GGC GCA CGT GAG ACA C 3' | (connexin 31.1) | (SEQ.ID.NO:10) |
| 5' TGA AGA CAA TGA AGA TGT T 3' | (connexin 31.1) | (SEQ.ID.NO:11) |
| 5' TTT CTT TTC TAT GTG CTG TTG GTG A 3' | (connexin 32) | (SEQ.ID.NO:12) |

Suitable polynucleotides for the preparation of the combined polynucleotide compositions described herein include for example, polynucleotides to Connexin Cx43 and polynucleotides for connexins 26, 30, 31.1, 32 and 37 as described in Table 1 above.

Although the precise sequence of the antisense polynucleotide used in the invention will depend upon the target connexin protein, for connexin 43, antisense polynucleotides having the following sequences have been found to be particularly suitable: GTA ATT GCG GCA AGA AGA ATT GTT TCT GTC (SEQ.ID.NO:1); GTA ATT GCG GCA GGA GGA ATT GTT TCT GTC (SEQ.ID.NO:2); and GGC AAG AGA CAC CAA AGA CAC TAC CAG CAT (SEQ.ID.NO:3).

For example, antisense polynucleotides for connexins 26, 31.1 and 32 have the following sequences:
5' TCC TGA GCA ATA CCT AAC GAA CAA ATA (connexin 26) (SEQ.ID.NO:4);
5' CGT CCG AGC CCA GAA AGA TGA GGT C (connexin 31.1) (SEQ.ID.NO:9); and
5' TTT CTT TTC TAT GTG CTG TTG GTG A (connexin 32) (SEQ.ID.NO:12).

Other connexin antisense polynucleotide sequences include:
5' CAT CTC CTT GGT GCT CAA CC 3' (connexin 37) (SEQ.ID.NO:5);
5' CTG AAG TCG ACT TGG CTT GG 3' (connexin 37) (SEQ.ID.NO:6);
5' CTC AGA TAG TGG CCA GAA TGC 3' (connexin 30) (SEQ.ID.NO:7);
5' TTG TCC AGG TGA CTC CAA GG 3' (connexin 30) (SEQ.ID.NO:8);
5' AGA GGC GCA CGT GAG ACA C 3' (connexin 31.1) (SEQ.ID.NO:10); and
5' TGA AGA CAA TGA AGA TGT T 3' (connexin 31.1) (SEQ.ID.NO:11).

Polynucleotides, including ODN's, directed to connexin proteins can be selected in terms of their nucleotide sequence by any convenient, and conventional, approach. For example, the computer programs MacVector and OligoTech (from Oligos etc. Eugene, Oregon, USA) can be used. Once selected, the ODN's can be synthesized using a DNA synthesizer.

### Polynucleotide Homologues

Homology and homologues are discussed herein (for example, the polynucleotide may be a homologue of a complement to a sequence in connexin mRNA). Such a polynucleotide typically has at least about 70% homology, preferably at least about 80%, at least about 90%, at least about 95%, at least about 97% or at least about 99% homology with the relevant sequence, for example over a region of at least about 15, at least about 20, at least about 40, at least about 100 more contiguous nucleotides (of the homologous sequence).

Homology may be calculated based on any method in the art. For example the UWGCG Package provides the BESTFIT program which can be used to calculate homology (for example used on its default settings) (Devereux et al (1984) Nucleic Acids Research 12, p387-395). The PILEUP and BLAST algorithms can be used to calculate homology or line up sequences (typically on their default settings), for example as described in Altschul S. F. (1993) J Mol Evol 36: 290-300; Altschul, S, F et al (1990) J Mol Biol 215: 403-10.

Software for performing BLAST analyses is publicly available through the National Center for Biotechnology Information (http://www.ncbi.nlm.nih.govn. This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul et al, supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached.

The BLAST algorithm parameters W, T and X determine the sensitivity and speed of the alignment. The BLAST program uses as defaults a word length (W), the BLOSUM62 scoring matrix (*see* Henikoff and Henikoff (1992) Proc. Natl. Acad. Sci. USA 89: 10915-10919) alignments (B) of 50, expectation (E) of 10, M=5, N=4, and a comparison of both strands.

The BLAST algorithm performs a statistical analysis of the similarity between two sequences; see *e.g.,* Karlin and Altschul (1993) Proc. Natl. Acad. Sci. USA 90: 5873-5787. One measure of similarity provided by the BLAST algorithm is the smallest sum probability (P(N)), which provides an indication of the probability by which a match between two nucleotide or amino acid sequences would occur by chance. For example, a sequence is considered similar to another sequence if the smallest sum probability in comparison of the first sequence to a second sequence is less than about 1, preferably less than about 0.1, more preferably less than about 0.01, and most preferably less than about 0.001.

The homologous sequence typically differs from the relevant sequence by at least about (or by no more than about) 2, 5, 10, 15, 20 more mutations (which may be substitutions, deletions or insertions). These mutations may be measured across any of the regions mentioned above in relation to calculating homology.

The homologous sequence typically hybridizes selectively to the original sequence at a level significantly above background. Selective hybridization is typically achieved using conditions of medium to high stringency (for example 0.03M sodium chloride and 0.03M sodium citrate at from about 50°C to about 60°C). However, such hybridization may be carried out under any suitable conditions known in the art (*see* Sambrook et al. (1989), Molecular Cloning: A Laboratory Manual). For example, if high stringency is required, suitable conditions include 0.2 x SSC at 60°C. If lower stringency is required, suitable conditions include 2 x SSC at 60°C.

### Peptide and Polypeptide Anti-Connexin Agents

Binding proteins, including peptides, peptidomimetics, antibodies, antibody fragments, and the like, are also suitable modulators of gap junctions and hemichannels.

Binding proteins include, for example, monoclonal antibodies, polyclonal antibodies, antibody fragments (including, for example, Fab, F(ab')₂ and Fv fragments; single chain antibodies; single chain Fvs; and single chain binding molecules such as those comprising, for example, a binding domain, hinge, CH2 and CH3 domains, recombinant antibodies and antibody fragments which are capable of binding an antigenic determinant (*i.e.,* that portion of a molecule, generally referred to as an epitope) that makes contact with a particular antibody or other binding molecule. These binding proteins, including antibodies, antibody fragments, and so on, may be chimeric or humanized or otherwise made to be less immunogenic in the subject to whom they are to be administered, and may be synthesized, produced recombinantly, or produced in expression libraries. Any binding molecule known in the art or later discovered is envisioned, such as those referenced herein and/or described in greater detail in the art. For example, binding proteins include not only antibodies, and the like, but also ligands, receptors, peptidomimetics, or other binding fragments or molecules (for example, produced by phage display) that bind to a target (*e.g.* connexin, hemichannel, or associated molecules).

Binding molecules will generally have a desired specificity, including but not limited to binding specificity, and desired affinity. Affinity, for example, may be a Kₐ of greater than or equal to about 10⁴ M⁻¹, greater than or equal to about 10⁶ M⁻¹, greater than or equal to about 10⁷ M⁻¹, greater than or equal to about 10⁸ M⁻¹. Affinities of even greater than about 10⁸ M⁻¹ are suitable, such as affinities equal to or greater than about 10⁹ M⁻¹, about 10¹⁰ M⁻¹, about 10¹¹ M⁻¹, and about 10¹² M⁻¹. Affinities of binding proteins according to the present invention can be readily determined using conventional techniques, for example those described by Scatchard et al., 1949 Ann. N.Y. Acad. Sci. 51: 660.

By using data obtained from hydropathy plots, it has been proposed that a connexin contains four-transmembrane-spanning regions and two short extra-cellular loops. The positioning of the first and second extracellular regions of connexin was further characterized by the reported production of anti-peptide antibodies used for immunolocalization of the corresponding epitopes on split gap junctions. Goodenough D.A. J Cell Biol 107: 1817-1824 (1988); Meyer R.A., J Cell Biol 119: 179-189 (1992).

The extracellular domains of a hemichannel contributed by two adjacent cells "dock" with each other to form complete gap junction channels. Reagents that interfere with the interactions of these extracellular domains can impair cell-to-cell communication. Peptide inhibitors of gap junctions and hemichannels have been reported. See for example Berthoud, V.M. et al., Am J. Physiol. Lung Cell Mol. Physiol. 279: L619 - L622 (2000); Evans, W.H. and Boitano, S. Biochem. Soc. Trans. 29: 606 - 612, and De Vriese A.S., et al. Kidney Int. 61: 177 - 185 (2001). Short peptides corresponding to sequences within the extracellular loops of connexins were said to inhibit intercellular communication. Boitano S. and Evans W. Am J Physiol Lung Cell Mol Physiol 279: L623-L630 (2000). The use of peptides as inhibitors of cell-cell channel formation produced by connexin (Cx) 32 expressed in paired *Xenopus* oocytes has also been reported. Dahl G, et al., Biophys J 67: 1816-1822 (1994). Berthoud, V.M. and Seul, K.H., summarized some of these results. Am J., Physiol. Lung Cell Mol. Physiol. 279: L619 - L622 (2000).

Anti-connexin agents include peptides comprising an amino acid sequence corresponding to a transmembrane region (*e.g.* 1^{st} to 4^{th}) of a connexin (*e.g.* connexin 45, 43, 26, 30, 31.1, and 37). Anti-connexin agents may comprise a peptide comprising an amino acid sequence corresponding to a portion of a transmembrane region of a connexin 45. Anti-connexin agents include a peptide having an amino acid sequence that comprises about 5 to 20 contiguous amino acids of SEQ.ID.NO:13, a peptide having an amino acid sequence that comprises about 8 to 15 contiguous amino acids of SEQ.ID.NO:13, or a peptide having an amino acid sequence that comprises about 11 to 13 contiguous amino acids of SEQ.ID.NO:13. Other embodiments are directed to an anti-connexin agent that is a peptide having an amino acid sequence that comprises at least about 5, at least about 6, at least about 7, at least about 8, at least about 9, at least about 10, at least about 11, at least about 12, at least about 13, at least about 14, at least about 15, at least about 20, at least about 25, or at least about 30 contiguous amino acids of SEQ.ID.NO:13. In certain anti-connexin agents provided herein, the extracellular domains of connexin 45 corresponding to the amino acids at positions 46-75 and 199-228 of SEQ ID NO: 13 may be used to develop the particular peptide sequences. Certain peptides described herein have an amino acid sequence corresponding to the regions at positions 46-75 and 199-228 of SEQ.ID.NO: 13. The peptides need not have an amino acid sequence identical to those portions of SEQ.ID.NO: 13, and conservative amino acid changes may be made such that the peptides retain binding activity or functional activity. Alternatively, the peptide may target regions of the connexin protein other than the extracellular domains (e.g. the portions of SEQ.ID.NO:13 not corresponding to positions 46-75 and 199-228).

Also, suitable anti-connexin agents comprise a peptide comprising an amino acid sequence corresponding to a portion of a transmembrane region of a connexin 43. Anti-connexin agents include peptides having an amino acid sequence that comprises about 5 to 20 contiguous amino acids of SEQ.ID.NO:14, peptides having an amino acid sequence that comprises about 8 to 15 contiguous amino acids of SEQ.ID.NO:14, or peptides having an amino acid sequence that comprises about 11 to 13 contiguous amino acids of SEQ.ID.NO: 14. Other anti-connexin agents include a peptide having an amino acid sequence that comprises at least about 5, at least about 6, at least about 7, at least about 8, at least about 9, at least about 10, at least about 11, at least about 12, at least about 13, at least about 14, at least about 15, at least about 20, at least about 25, or at least about 30 contiguous amino acids of SEQ.ID.NO:14. Other anti-connexin agents comprise the extracellular domains of connexin 43 corresponding to the amino acids at positions 37-76 and 178-208 of SEQ.ID.NO: 14. Anti-connexin agents include peptides described herein which have an amino acid sequence corresponding to the regions at positions 37-76 and 178-208 of SEQ.ID.NO: 14. The peptides need not have an amino acid sequence identical to those portions of SEQ.ID.NO: 14, and conservative amino acid changes may be made such that the peptides retain binding activity or functional activity. Alternatively, peptides may target regions of the connexin protein other than the extracellular domains (*e.g.* the portions of SEQ.ID.NO:14 not corresponding to positions 37-76 and 178-208).
**Connexin 45 (SEQ ID NO**.**13**) 
Met Ser Trp Ser Phe Leu Thr Arg Leu Leu Glu Glu Ile His Asn His
1               5                   10                  15

Ser Thr Phe Val Gly Lys Ile Trp Leu Thr Val Leu Ile Val Phe Arg
            20                  25                  30

Ile Val Leu Thr Ala Val Gly Gly Glu Ser Ile Tyr Tyr Asp Glu Gln
        35                  40                  45

Ser Lys Phe Val Cys Asn Thr Glu Gln Pro Gly Cys Glu Asn Val Cys
    50                  55                  60

Tyr Asp Ala Phe Ala Pro Leu Ser His Val Arg Phe Trp Val Phe Gln
65                  70                  75                  80

Ile Ile Leu Val Ala Thr Pro Ser Val Met Tyr Leu Gly Tyr Ala Ile
                85                  90                  95

His Lys Ile Ala Lys Met Glu His Gly Glu Ala Asp Lys Lys Ala Ala
            100                 105                 110

Arg Ser Lys Pro Tyr Ala Met Arg Trp Lys Gln His Arg Ala Leu Glu
        115                 120                 125

Glu Thr Glu Glu Asp Asn Glu Glu Asp Pro Met Met Tyr Pro Glu Met
    130                 135                 140

Glu Leu Glu Ser Asp Lys Glu Asn Lys Glu Gln Ser Gln Pro Lys Pro
145                 150                 155                 160

Lys His Asp Gly Arg Arg Arg Ile Arg Glu Asp Gly Leu Met Lys Ile
                165                 170                 175

Tyr Val Leu Gln Leu Leu Ala Arg Thr Val Phe Glu Val Gly Phe Leu
            180                 185                 190

Ile Gly Gln Tyr Phe Leu Tyr Gly Phe Gln Val His Pro Phe Tyr Val
        195                 200                 205

Cys Ser Arg Leu Pro Cys Pro His Lys Ile Asp Cys Phe Ile Ser Arg
    210                 215                 220
 
Pro Thr Glu Lys Thr Ile Phe Leu Leu Ile Met Tyr Gly Val Thr Gly
225                 230                 235                 240

Leu Cys Leu Leu Leu Asn Ile Trp Glu Met Leu His Leu Gly Phe Gly
                245                 250                 255

Thr Ile Arg Asp Ser Leu Asn Ser Lys Arg Arg Glu Leu Glu Asp Pro
            260                 265                 270

Gly Ala Tyr Asn Tyr Pro Phe Thr Trp Asn Thr Pro Ser Ala Pro Pro
        275                 280                 285

Gly Tyr Asn Ile Ala Val Lys Pro Asp Gln Ile Gln Tyr Thr Glu Leu
    290                 295                 300

Ser Asn Ala Lys Ile Ala Tyr Lys Gln Asn Lys Ala Asn Thr Ala Gln
305                 310                 315                 320

Glu Gln Gln Tyr Gly Ser His Glu Glu Asn Leu Pro Ala Asp Leu Glu
                325                 330                 335

Ala Leu Gln Arg Glu Ile Arg Met Ala Gln Glu Arg Leu Asp Leu Ala
            340                 345                 350

Val Gln Ala Tyr Ser His Gln Asn Asn Pro His Gly Pro Arg Glu Lys
        355                 360                 365

Lys Ala Lys Val Gly Ser Lys Ala Gly Ser Asn Lys Ser Thr Ala Ser
    370                 375                 380

Ser Lys Ser Gly Asp Gly Lys Asn Ser Val Trp Ile
385                 390                 395
**Connexin 43 (SEQ ID NO. 14)**


1               5                   10                  15


            20                  25                  30


        35                  40                  45


    50                  55                  60


65                  70                  75                  80


                85                  90                  95


            100                 105                 110


        115                 120                 125



130                 135                 140


145                 150                 155                 160


                165                 170                 175


            180                 185                 190


        195                 200                 205


    210                 215                 220


225                 230                 235                 240


                245                 250                 255


            260                 265                 270


        275                 280                 285


    290                 295                 300


305                 310                 315                 320


                325                 330                 335


            340                 345                 350


        355                 360                 365


    370                 375                 380

The anti-connexin peptides may comprise sequences corresponding to a portion of the connexin extracellular domains with conservative amino acid substitutions such that peptides are functionally active anti-connexin agents. Exemplary conservative amino acid substitutions include for example the substitution of a nonpolar amino acid with another nonpolar amino acid, the substitution of an aromatic amino acid with another aromatic amino acid, the substitution of an aliphatic amino acid with another aliphatic amino acid, the substitution of a polar amino acid with another polar amino acid, the substitution of an acidic amino acid with another acidic amino acid, the substitution of a basic amino acid with another basic amino acid, and the substitution of an ionizable amino acid with another ionizable amino acid.

Exemplary peptides targeted to connexin 43 are shown below in Table 2. M1, 2, 3 and 4 refer to the 1^{st} to 4^{th} transmembrane regions of the connexin 43 protein respectively. E1 and E2 refer to the first and second extracellular loops respectively.

**Table 2. Peptidic Inhibitors of Intercellular Communication (cx43)**

| | | |
|---|---|---|
| FEVAFLLIQWI | M3 & E2 | (SEQ.ID.NO:15) |
| LLIQWYIGFSL | E2 | (SEQ.ID.NO:16) |
| SLSAVYTCKRDPCPHQ | E2 | (SEQ.ID.NO:17) |
| VDCFLSRPTEKT | E2 | (SEQ.ID.NO:18) |
| SRPTEKTIFII | E2 & M4 | (SEQ.ID.NO:19) |
| LGTAVESAWGDEQ | M1 & E1 | (SEQ.ID.NO:20) |
| QSAFRCNTQQPG | E1 | (SEQ.ID.NO:21) |
| QQPGCENVCYDK | E1 | (SEQ.ID.NO:22) |
| VCYDKSFPISHVR | E1 | (SEQ.ID.NO:23) |

Table 3 provides additional exemplary connexin peptides used in inhibiting hemichannel or gap junction function. In other embodiments, conservative amino acid changes are made to the peptides or fragments thereof.

**Table 3. Additional Peptidic Inhibitors of Intercellular Communication (cx32, cx43)**

| Connexin | Location | | AA's and Sequence |
|---|---|---|---|
| Cx32 | E1 39-77 | AAESVWGDEIKSSFICNTLQPGCNSVCYDHFFPIS HVR | (SEQ.ID.NO:24) |
| Cx32 | E1 41-52 | ESVWGDEKSSFI | (SEQ.ID.NO:25) |
| Cx32 | E1 52-63 | ICNTLQPGCNSV | (SEQ.ID.NO:26) |
| Cx32 | E1 62-73 | SVCYDHFFPISH | (SEQ.ID.NO:27) |
| Cx32 | E2 64-188 | RLVKCEAFPCPNTVDCFVSRPTEKT | (SEQ.ID.NO:28) |
| Cx32 | E2 166-177 | VKCEAFPCPNTV | (SEQ.ID.NO:29) |
| Cx32 | E2 177-188 | VDCFVSRPTEKT | (SEQ.ID.NO:30) |
| Cx32 | E1 63-75 | VCYDHFFPISHVR | (SEQ.ID.NO:31) |
| Cx32 | E1 45-59 | VWGDEKSSFICNTLQPGY | (SEQ.ID.NO:32) |
| Cx32 | E1 46-59 | DEKSSFICNTLQPGY | (SEQ.ID.NO:33) |
| Cx32 | E2 182-192 | SRPTEKTVFTV | (SEQ.ID.NO:34) |
| Cx32/Cx43 | E2 182-188/ 201-207 | SRPTEKT | (SEQ.ID.NO:35) |
| Cx32 | E1 52-63 | ICNTLQPGCNSV | (SEQ.ID.NO:36) |
| Cx40 | E2 177-192 | FLDTLHVCRRSPCPHP | (SEQ.ID.NO:37) |
| Cx43 | E2 188-205 | KRDPCHQVDCFLSRPTEK | (SEQ.ID.NO:38) |

Table 4 provides the extracellular loops for connexin family members which are used to develop peptide inhibitors for use as described herein. The peptides and provided in Table 4, and fragments thereof, are used as peptide inhibitors in certain non-limiting embodiments. In other non-limiting embodiments, peptides comprising from about 8 to about 15, or from about 11 to about 13 amino contiguous amino acids of the peptides in this Table 4 are peptide inhibitors. Conservative amino acid changes may be made to the peptides or fragments thereof.

**Table 4. Extracellular loops for various connexin family members**

| **E1** | | |
|---|---|---|
| huCx26 | KEVWGDEQADFVCNTLQPGCKNVCYDHYFPISHIR | (SEQ.ID.NO:39) |
| huCx30 | QEVWGDEQEDFVCNTLQPGCKNVCYDHFFPVSHIR | (SEQ.ID.NO:40) |
| huCx30.3 | EEVWDDEQKDFVCNTKQPGCPNVCYDEFFPVSHVR | (SEQ.ID.NO:41) |
| huCx31 | ERVWGDEQKDFDCNTKQPGCTNVCYDNYFPISNIR | (SEQ.ID.NO:42) |
| huCx31.1 | ERVWSDDHKDFDCNTRQPGCSNVCFDEFFPVSHVR | (SEQ.ID.NO:43) |
| huCx32 | ESVWGDEKSSFICNTLQPGCNSVCYDQFFPISHVR | (SEQ.ID.NO:44) |
| huCx36 | ESVWGDEQSDFECNTAQPGCTNVCYDQAFPISHIR | (SEQ.ID.NO:45) |
| huCx37 | ESVWGDEQSDFECNTAQPGCTNVCYDQAFPISHIR | (SEQ.ID.NO:46) |
| huCx40.1 | RPVYQDEQERFVCNTLQPGCANVCYDVFSPVSHLR | (SEQ.ID.NO:47) |
| huCx43 | ESAWGDEQSAFRCNTQQPGCENVCYDKSFPISHVR | (SEQ.ID.NO:48) |
| huCx46 | EDVWGDEQSDFTCNTQQPGCBNVCYBRAFPISHIR | (SEQ.ID.NO:49) |
| huCx46.6 | EAIYSDEQAKFTCNTRQPGCDNVCYDAFAPLSHVR | (SEQ.ID.NO:50) |
| huCx40 | ESSWGDEQADFRCDTIQPGCQNVCTDQAFPISHIR | (SEQ.ID.NO:51) |
| huCx45 | GESIYYDEQSKFVCNTEQPGCENVCYDAFAPLSHVR | (SEQ.ID.NO:52) |
| **E2** | | |
| huCx26 | MYVFYVMYDGFSMQRLVKCNAWPCPNTVDCFVSRPTEKT | (SEQ.ID.NO:53) |
| huCx30 | MYVFYFLYNGYHLPWVLKCGIDPCPNLVDCFISRPTEKT | (SEQ.ID.NO:54) |
| huCx30.3 | LYIFHRLYKDYDMPRVVACSVEPCPHTVDCYISRPTEKK | (SEQ.ID.NO:55) |
| huCx31 | LYLLHTLWHGFNMPRLVQCANVAPCPNIVDCYIARPTEKK | (SEQ.ID.NO:56) |
| huCx31.1 | LYVFHSFYPKYILPPVVKCHADPCPNIVDCFISKPSEKN | (SEQ.ID.NO:57) |
| huCx32 | MYVFYLLYPGYAMVRLVKCDVYPCPNTVDCFVSRPTEKT | (SEQ.ID.NO:58) |
| huCx36 | LYGWTMEPVFVCQRAPCPYLVDCFVSRPTEKT | (SEQ.ID.NO:59) |
| huCx37 | LYGWTMEPVFVCQRAPCPYLVDCFVSRPTEKT | (SEQ.ID.NO:60) |
| huCx40.1 | GALHYFLFGFLAPKKFPCTRPPCTGVVDCYVSRPTSKS | (SEQ.ID.NO:61) |
| huCx43 | LLIQWYIYGFSLSAVYTCKRDPCPHQVDCFLSRPTEKT | (SEQ.ID.NO:62) |
| huCx46 | IAGQYFLYGFELKPLYRCDRWPCPNTVDCFISRPTEKT | (SEQ.ID.NO:63) |
| huCx46.6 | LVGQYLLYGFEVRPFFPCSRQPCPHVVDCFVSRPTEKT | (SEQ.ID.NO:64) |
| huCx40 | IVGQYFIYGIFLTTLHVCRRSPCPHPVNCYVSRPTEKN | (SEQ.ID.NO:65) |
| huCx45 | LIGQYFLYGFQVHPFYVCSRLPCHPKIDCFISRPTEKT | (SEQ.ID.NO:66) |

Table 5 provides the extracellular domain for connexin family members which may be used to develop peptide anti-connexin agents. The peptides and provided in Table 5, and fragments thereof, may also be used as peptide anti-connexin agents. Such peptides may comprise from about 8 to about 15, or from about 11 to about 13 amino contiguous amino acids of the peptide sequence in this Table 5. Conservative amino acid changes may be made to the peptides or fragments thereof.

**Table 5. Extracellular domains**

| | | |
|---|---|---|
| Peptide | VDCFLSRPTEKT | (SEQ.ID.NO:18) |
| Peptide | SRPTEKTIFII | (SEQ.ID.NO:19) |
| huCx43· | LLIQWYIYGFSLSAVYTCKRDPCPHQVDCFLSRPTEKTIFII | (SEQ.ID.NO:67) |
| huCx26 | MYVFYVMYDGFSMQRLVKCNAWPCPNTVDCFVSRPTEKTVFTV | (SEQ.ID.NO:68) |
| huCx30 | YVFYFLYNGYHLPWVLKCGIDPCPNLV DCFISRPTEKTVFTI | (SEQ.ID.NO:69) |
| huCx30.3 | LYIFHRLYKDYDMPRVVACSVEPCPHTVDCYISRPTEKKVFTY | (SEQ.ID.NO:70) |
| huCx31 | LYLLHTLWHGFNMPRLVQCANVAPCPNIVDCYIARPTEKKTY | (SEQ.ID.NO:71) |
| huCx31.1 | LYVFHSFYPKYILPPVVKCHADPCPNIVDCFISKPSEKNIFTL | (SEQ.ID.NO:72) |
| huCx32 | MYVFYLLYPGYAMVRLVKCDVYPCPNTVDCFVSRPTEKTVFTV | (SEQ.ID.NO:73) |
| huCx36 | LYGWTMEPVFVCQRAPCPYLVDCFV SRPTEKTIFII | (SEQ.ID.NO:74) |
| huCx37 | LYGWTMEPVFVCQRAPCPYLVDCFVSRPTEKTIFII | (SEQ.ID.NO:75) |
| huCx40.1 | GALHYFLFGFLAPKKFPCTRPPCTGVVDCYVSRPTEKSLLML | (SEQ.ID.NO:76) |
| huCx46 | IAGQYFLYGFELKPLYRCDRWPCPNTVDCFISRPTEKTIFII | (SEQ.ID.NO:77) |
| huCx46.6 | LVGQYLLYGFEVRPFFPCSRQPCPHVVDCFVSRPTEKTVFLL | (SEQ.ID.NO:78) |
| huCx40 | NGQYFIYGIFLTTLHVCRRSPCPHPVNCYSRPTEKNVFIV | (SEQ.ID.NO:79) |
| huCx45 | LIGQYFLYGFQVHPFYVCSRLPCHPKIDCFISRPTEKTIFLL | (SEQ.ID.N0:80) |

Table 6 provides peptides inhibitors of connexin 40 shown with reference to the extracellular loops (E1 and E2) of connexin 40. The bold amino acids are directed to the transmembrane regions of connexin 40.

**Table 6. Cx40 peptide inhibitors**

| **E2** | | |
|---|---|---|
| | **LGTAA**ESSWGDEQADFRCDTIQPGCQNVCTDQAFPISHIR**FWVLQ** | (SEQ.ID.NO:81) |
| | LGTAAESSWGDEQA | (SEQ.ID.NO:82) |
| | DEQADFRCDTIQP | (SEQ.ID.NO:83) |
| | TIQPGCQNVCTDQ | (SEQ.ID.NO:84) |
| | VCTDQAFPISHIR | (SEQ.ID.NO:85) |
| | AFPISHIRFWVLQ | (SEQ.ID.NO:86) |

| **E2** | | |
|---|---|---|
| | **MEVGF**IVGQYFIYGIFLTTLHVCRRSPCPHPVNCYVSRPTEKN**VFIV** | (SEQ.ID.NO:87) |
| | MEVGFIVGQYF | (SEQ.ID.NO:88) |
| | IVGQYFIYGIFL | (SEQ.ID.NO:89) |
| | GIFLTTLHVCRRSP | (SEQ.ID.NO:90) |
| | RRSPCPHPVNCY | (SEQ.ID.NO:91) |
| | **VNCYVSRPTEKN** | (SEQ.ID.NO:92) |
| | **SRPTEKNVFIV** | (SEQ.ID.NO:93) |

Table 7 provides peptides inhibitors of connexin 45 shown with reference to the extracellular loops (E1 and E2) of connexin 45. The bold amino acids are directed to the transmembrane regions of connexin 45

**Table 7. Cx45 peptide inhibitors**

| **E1** | | |
|---|---|---|
| | **LTAVG**GESIYYDEQSKFVCNTEQPGCENVCYDAFAPLSHVR**FWVFQ** | (SEQ.ID.NO:94) |
| | **LTAVG**GESIYYDEQS | (SEQ.ID.NO:95) |
| | DEQSKFVCNTEQP | (SEQ.ID.NO:96) |
| | TEQPGCENVCYDA | (SEQ.ID.NO:97) |
| | VCYDAFAPLSHVR | (SEQ.ID.NO:98) |
| | APLSHVRFWVFQ | (SEQ.ID.NO:99) |

| **E2** | | |
|---|---|---|
| | **FEVGF**LIGQYFLYGFQVHPFYVCSRLPCHPKIDCFISRPTEKT**IFLL** | (SEQ.ID.NO:100) |
| | FEVGFLIGQYF | (SEQ.ID.NO:101) |
| | LIGQYFLYGFQV | (SEQ.ID.NO:102) |
| | GFQVHPFYVCSRLP | (SEQ.ID.NO:103) |
| | SRLPCHPKIDCF | (SEQ.ID.NO:104) |
| | **IDCFISRPTEKT** | (SEQ.ID.NO:105) |
| | **SRPTEKTIFLL** | (SEQ.ID.NO:106) |

In certain embodiments, it is preferred that certain peptide inhibitors block hemichannels without disrupting existing gap junctions. While not wishing to be bound to any particular theory or mechanism, it is also believed that certain peptidomimetics (*e.g.* VCYDKSFPISHVR, (SEQ.ID.NO: 23) block hemichannels without causing uncoupling of gap junctions (*See* Leybeart et al., Cell Commun. Adhes. 10: 251-257 (2003)), or do so in lower dose amounts. The peptide SRPTEKTIFII (SEQ.ID.NO: 19) may also be used, for example to block hemichannels without uncoupling of gap junctions. The peptide SRGGEKNVFIV (SEQ.ID.NO: 107) may be used that as a control sequence (DeVriese et al., Kidney Internat. 61: 177-185 (2002)). Examples of peptide inhibitors for connexin 45 YVCSRLPCHP (SEQ.ID.NO:108), QVHPFYVCSRL (SEQ.ID.NO:109), FEVGFLIGQYFLY (SEQ.ID.NO:110), GQYFLYGFQVHP (SEQ.ID.NO:111), GFQVHPFYVCSR (SEQ.ID.NO:112), AVGGESIYYDEQ (SEQ.ID.NO.:113), YDEQSKFVCNTE (SEQ.ID.NO:114), NTEQPGCENVCY (SEQ.ID.NO:115), CYDAFAPLSHVR (SEQ.ID.NO:116), FAPLSHVRFWVF (SEQ.ID.NO:117) and LIGQY (SEQ.ID.NO:118), QVHPF (SEQ.ID.NO:119), YVCSR (SEQ.ID.NO:120), SRLPC (SEQ.ID.NO:121), LPCHP (SEQ.ID.NO:122) and GESIY (SEQ.ID.NO:123), YDEQSK (SEQ.ID.NO:124), SKFVCN (SEQ.ID.NO:125), TEQPGCEN (SEQ.ID.NO:126), VCYDAFAP (SEQ.ID.NO:127), LSHVRFWVFQ (SEQ.ID.NO:128) The peptides may only be 3 amino acids in length, including SRL, PCH, LCP, CHP, IYY, SKF, QPC, VCY, APL, HVR, or longer, for example: LIQYFLYGFQVHPF (SEQ.ID.NO:129), VHPFYCSRLPCHP (SEQ.ID.NO:130), VGGESIYYDEQSKFVCNTEQPG (SEQ.ID.NO:131), TEQPGCENVCYDAFAPLSHVRF (SEQ.ID.NO:132), AFAPLSHVRFWVFQ (SEQ.ID.NO: 133).

**Table 8**

| Table 8A | |
|---|---|
| Human Connexin 43 from GenBank Accession No. M65188 (SEQ.ID.NO:134) | |
| 1 | ggcttttagc gtgaggaaag taccaaacag cagcggagtt ttaaacttta aatagacagg |
| 61 | tctgagtgcc tgaacttgcc ttttcatttt acttcatcct ccaaggagtt caatcacttg |
| 121 | gcgtgacttc actactttta agcaaaagag tggtgcccag gcaacatggg tgactggagc |
| 181 | gccttaggca aactccttga caaggttcaa gcctactcaa ctgctggagg gaaggtgtgg |
| 241 | ctgtcagtac ttttcatttt ccgaatcctg ctgctgggga cagcggttga gtcagcctgg |
| 301 | ggagatgagc agtctgcctt tcgttgtaac actcagcaac ctggttgtga aaatgtctgc |
| 361 | tatgacaagt ctttcccaat ctctcatgtg cgcttctggg tcctgcagat catatttgtg |
| 421 | tctgtaccca cactcttgta cctggctcat gtgttctatg tgatgcgaaa ggaagagaaa |
| 481 | ctgaacaaga aagaggaaga actcaaggtt gcccaaactg atggtgtcaa tgtggacatg |
| 541 | cacttgaagc agattgagat aaagaagttc aagtacggta ttgaagagca tggtaaggtg |
| 601 | aaaatgcgag gggggttgct gcgaacctac atcatcagta tcctcttcaa gtctatcttt |
| 661 | gaggtggcct tcttgctgat ccagtggtac atctatggat tcagcttgag tgctgtttac |
| 721 | acttgcaaaa gagatccctg cccacatcag gtggactgtt tcctctctcg ccccacggag |
| 781 | aaaaccatct tcatcatctt catgctggtg gtgtccttgg tgtccctggc cttgaatatc |
| 841 | attgaactct tctatgtttt cttcaagggc gttaaggatc gggttaaggg aaagagcgac |
| 901 | ccttaccatg cgaccagtgg tgcgctgagc cctgccaaag actgtgggtc tcaaaaatat |
| 961 | gcttatttca atggctgctc ctcaccaacc gctcccctct cgcctatgtc tcctcctggg |
| 1021 | tacaagctgg ttactggcga cagaaacaat tcttcttgcc gcaattacaa caagcaagca |
| 1081 | agtgagcaaa actgggctaa ttacagtgca gaacaaaatc gaatggggca ggcgggaagc |
| 1141 | accatctcta actcccatgc acagcctttt gatttccccg atgataacca gaattctaaa |
| 1201 | aaactagctg ctggacatga attacagcca ctagccattg tggaccagcg accttcaagc |
| 1261 | agagccagca gtcgtgccag cagcagacct cggcctgatg acctggagat ctag |

| Table 8B | |
|---|---|
| Human Connexin 43 (SEQ.ID.NO:135) | |
| 1 | atgggtgactggagcgcctt aggcaaactc cttgacaagg ttcaagccta ctcaactgct |
| 61 | ggagggaaggtgtggctgtc agtacttttc attttccgaatcctgctgct ggggacagcg |
| 121 | gttgagtcagcctggggaga tgagcagtct gcctttcgtt gtaacactca gcaacctggt |
| 181 | tgtgaaaatg tctgctatga caagtctttcccaatctctc atgtgcgctt ctgggtcctg |
| 241 | cagatcatat ttgtgtctgt acccacactcttgtacctgg ctcatgtgttctatgtgatg |
| 301 | cgaaaggaag agaaactgaa caagaaagag gaagaactca aggttgccca aactgatggt |
| 361 | gtcaatgtgg acatgcactt gaagcagatt gagataaagaagttcaagta cggtattgaa |
| 421 | gagcatggta aggtgaaaat gcgagggggg ttgctgcgaa cctacatcat cagtatcctc |
| 481 | ttcaagtcta tctttgaggt ggccttcttg ctgatccagt ggtacatcta tggattcagc |
| 541 | ttgagtgctg tttacacttg caaaagagat ccctgcccac atcaggtgga ctgtttcctc |
| 601 | tctcgcccca cggagaaaac catcttcatc atcttcatgc tggtggtgtc cttggtgtcc |
| 661 | ctggccttga atatcattga actcttctat gttttcttca agggcgttaa ggatcgggtt |
| 721 | aagggaaaga gcgaccctta ccatgcgacc agtggtgcgc tgagccctgc caaagactgt |
| 781 | gggtctcaaa aatatgctta tttcaatggc tgctcctcac caaccgctcc cctctcgcct |
| 841 | atgtctcctc ctgggtacaa gctggttact ggcgacagaa acaattcttc ttgccgcaat |
| 901 | tacaacaagc aagcaagtga gcaaaactgg gctaattaca gtgcagaaca aaatcgaatg |
| 961 | gggcaggcgg gaagcaccat ctctaactcc catgcacagccttttgattt ccccgatgat |
| 1021 | aaccagaatt ctaaaaaactagctgctgga catgaattac agccactagc cattgtggac |
| 1081 | cagcgacctt caagcagagc cagcagtcgtgccagcagca gacctcggcctgatgacctg |
| 1141 | gagatctag |

### Therapeutic Agents

Therapeutic agents include pharmaceutically acceptable agents useful in the treatment of wounds or the promotion of wound-healing, whether currently existing and known or later developed. Therapeutic agents include, for example, anti-infectives, anesthetics, analgesics, antibiotics, narcotics, and steroidal and non-steroidal anti-inflammatory agents. Preferred therapeutic agents include topical steroid anti-inflammatory agents, antimicrobial agents, local and topical anesthetics, and topical opioids. In certain embodiments, one, two three, four, five or six therapeutic agents may be used in combination.

### Agents Useful for Wound Healing

As used herein, agents useful for wound healing include stimulators, enhancers or positive mediators of the wound healing cascade which 1) promote or accelerate the natural wound healing process or 2) reduce effects associated with improper or delayed wound healing, which effects include, for example, adverse inflammation, epithelialization, angiogenesis and matrix deposition, and scarring and fibrosis.

Positive mediators, enhancers and stimulators include for example, an agent which may stimulate, enhance, facilitate, or accelerate (*i.e.*, agonize) the quantity, quality or efficacy of wound healing or the active wound healing process, or a wound healing-associated growth factor or cytokine at a wound site, or the activation of a wound healing-associated growth factor or cytokine receptor. Such agents may include a wound healing-associated growth factor or cytokine or a partially modified form of a wound healing-associated growth factor or cytokine, for example. A partially modified form of wound healing-associated growth factor or cytokine may, for example, have a longer half-life than the natural wound healing-associated growth factor or cytokine. Alternatively, it may be an inhibitor of wound healing-associated growth factor or cytokine metabolism.

Partial modification of such an agent may be by way of addition, deletion or substitution of amino acid residues. A substitution may for example be a conserved substitution. Hence a partially modified molecule may be a homologue of the molecule from which it was derived. It may have at least about 40%, for example about 50, 60, 70, 80, 90 or 95%, homology with the molecule from which it is derived.

As used herein, agents useful for wound healing may include for example, wound-healing-promoting or scar-reducing agents for wound treatment modalities now known in the art or later-developed; exemplary factors, agents or modalities including natural or synthetic growth factors, cytokines, or modulators thereof to promote wound healing, wound healing promoting bioengineered matrix, dressings, bandages, and the like. Suitable examples may include, but not limited to **1)** antimicrobial agents, including systemic or topical creams or gels, including, for example, silver-containing agents such as SAGs (silver antimicrobial gels), (CollaGUARD(TM), Innocoll, Inc) (purified type-I collagen protein based dressing), CollaGUARD Ag (a collagen-based bioactive dressing impregnated with silver for infected wounds or wounds at risk of infection), DermaSIL(TM) (a collagen-synthetic foam composite dressing for deep and heavily exuding wounds); **2)** cell therapy, 3) cytokines, growth factors or hormones (both natural and synthetic) introduced to the wound to promote wound healing, including, for example, NGF, NT3, BDGF, integrins, plasmin, semaphoring, blood-derived growth factor, keratinocyte growth factor, tissue growth factor, TGF-alpha, TGF-beta, PDGF (one or more of the three subtypes may be used: AA, AB, and B), PDGF-BB, TGF-beta 3, factors that modulate the relative levels of TGFβ3, TGFβ1, and TGFβ2 (*e.g.,* Mannose-6-phosphate), sex steroids, including for example, estrogen, estradiol, or an oestrogen receptor agonist selected from the group consisting of ethinyloestradiol, dienoestrol, mestranol, oestradiol, oestriol, a conjugated oestrogen, piperazine oestrone sulphate, stilboestrol, fosfesterol tetrasodium, polyestradiol phosphate, tibolone, a phytoestrogen, 17-beta-estradiol; thymic hormones such as Thymosin-beta-4, EGF, HB-EGF, fibroblast growth factors (*e.g.,* FGF1, FGF2, FGF7), keratinocyte growth factor, TNF, interleukins family of inflammatory response modulators such as, for example, IL-10, IL-1, IL-2, IL-6, IL-8, and IL-10 and modulators thereof; INFs (INF-alpha, -beta, and -delta); stimulators of activin or inhibin, and inhibitors of interferon gamma prostaglandin E2 (PGE2) and of mediators of the adenosine 3',5'-cyclic monophosphate (cAMP) pathway; adenosine A1 agonist, adenosine A2 agonist or **5)** other agents useful for wound healing, including, for example, both natural or synthetic homologues, agonist and antagonist of VEGF, VEGFA, IGF; IGF-1, proinflammatory cytokines, GM-CSF, and leptins and 6) IGF-1 and KGF cDNA, autologous platelet gel, hypochlorous acid (Sterilox® lipoic acid, nitric oxide synthase3, matrix metalloproteinase 9 (MMP-9), CCT-ETA, alphavbeta6 integrin, growth factor-primed fibroblasts and Decorin, silver containing wound dressings, Xenaderm™, papain wound debriding agents, lactoferrin, substance P, collagen, and silver-ORC, placental alkaline phosphatase or placental growth factor, modulators of hedgehog signaling, modulators of cholesterol synthesis pathway, and APC (Activated Protein C), keratinocyte growth factor, TNF, Thromboxane A2, NGF, BMP bone morphogenetic protein, CTGF (connective tissue growth factor), wound healing chemokines, decorin, modulators of lactate induced neovascularization, cod liver oil, placental alkaline phosphatase or placental growth factor, and thymosin beta 4. In certain embodiments, one, two three, four, five or six agents useful for wound healing may be used in combination.

It is to be understood that the agents useful for wound healing (including for example, growth factors and cytokines) above encompass all naturally occurring polymorphs (for example, polymorphs of the growth factors or cytokines). Also, functional fragments, chimeric proteins comprising one of said agents useful for wound healing or a functional fragment thereof, homologues obtained by analogous substitution of one or more amino acids of the wound healing agent, and species homologues are encompassed. It is contemplated that one or more agents useful for wound healing may be a product of recombinant DNA technology, and one or more agents useful for wound healing may be a product of transgenic technology. For example, platelet derived growth factor may be provided in the form of a recombinant PDGF or a gene therapy vector comprising a coding sequence for PDGF.

A fragment or partially modified form thereof refers to a fragment or partially modified form of the wound healing agent which retains the biological or wound healing functionality of the factor, although it may of course have additional functionality. Partial modification may, for example, be by way of addition, deletion or substitution of amino acid residues. For example, a substitution may be a conserved substitution. Hence the partially modified molecules may be homologues of the wound healing agent. They may, for example, have at least about 40% homology with said factor. They may for example have at least about 50, 60, 70, 80, 90 or 95% homology with said factor. For example, in certain embodiments, IL-10 or a fragment or a partially modified form thereof may be administered at a concentration of between about 1 µM and about 10 µM. It may be administered at a concentration of between about 2.5 µM and about 5 µM. In certain other embodiments, IL-10 or a fragment or a partially modified form thereof may be administered immediately prior to wound healing, but may be effective if administered within about 7 days of wounding. It could be administered on at least two occasions.

### Anti-Microtubule Agents

Exemplary anti-microtubule agents include, for example, diterpenoids (*e.g.* paclitaxel, docetaxel, and derivatives or analogues thereof) and vinca alkaloids (*e.g.* vinblastine, vincristine, and vinorelbine); platinum coordination complexes (*e.g.* cisplatin and carboplatin).

### Dosage Formulation and Administration

The anti-connexin agents (including ODN's) of the invention (typically in the form of the formulations discussed herein) may be administered to a subject in need of treatment, such as a subject with any of the diseases or conditions mentioned herein. The condition of the subject can thus be improved. The anti-connexin agent and formulation may thus be used in the treatment of the subject's body by therapy. They may be used in the manufacture of a medicament to treat any of the conditions mentioned herein.

Thus, in accordance with the invention, there are provided formulations by which cell-cell communication can be downregulated in a transient and site-specific manner. The anti-connexin agent may be conveniently formulated with a pharmaceutically acceptable carrier to give the desired final concentration.

The anti-connexin agent may be present in a substantially isolated form. It will be understood that the product may be mixed with carriers or diluents which will not interfere with the intended purpose of the product and still be regarded as substantially isolated. A product of the invention may also be in a substantially purified form, in which case it will generally comprise about 80%, 85% or 90%, *e.g.* at least about 95%, at least about 98% or at least about 99% of the polynucleotide (or other anti-connexin agent) or dry mass of the preparation.

Depending on the intended route of administration, the pharmaceutical products, pharmaceutical compositions, combined preparations and medicaments of the invention may, for example, take the form of solutions, suspensions, instillations, salves, creams, gels, foams, ointments, emulsions, lotions, paints, sustained release formulations, or powders, and typically contain .01% to about 1% of active ingredient(s), about 1 %-50% or active ingredient(s), about 2%-60% of active ingredient(s), about 2%-70% of active ingredient(s), or up to about 90% of active ingredient(s). Other suitable formulations include pluronic gel-based formulations, carboxymethylcellulose(CMC)-based formulations, and hyroxypropylmethylcellulose(HPMC)-based formulations. Other useful formulations include slow or delayed release preparations.

Gels or jellies may be produced using a suitable gelling agent including, but not limited to, gelatin, tragacanth, or a cellulose derivative and may include glycerol as a humectant, emollient, and preservative. Ointments are semi-solid preparations that consist of the active ingredient incorporated into a fatty, waxy, or synthetic base. Examples of suitable creams include, but are not limited to, water-in-oil and oil-in-water emulsions. Water-in-oil creams may be formulated by using a suitable emulsifying agent with properties similar, but not limited, to those of the fatty alcohols such as cetyl alcohol or cetostearyl alcohol and to emulsifying wax. Oil-in-water creams may be formulated using an emulsifying agent such as cetomacrogol emulsifying wax. Suitable properties include the ability to modify the viscosity of the emulsion and both physical and chemical stability over a wide range of pH. The water soluble or miscible cream base may contain a preservative system and may also be buffered to maintain an acceptable physiological pH.

Foam preparations may be formulated to be delivered from a pressurized aerosol canister, via a suitable applicator, using inert propellants. Suitable excipients for the formulation of the foam base include, but are not limited to, propylene glycol, emulsifying wax, cetyl alcohol, and glyceryl stearate. Potential preservatives include methylparaben and propylparaben.

Preferably the anti-connexin agents are combined with a pharmaceutically acceptable carrier or diluent to produce a pharmaceutical composition. Suitable carriers and diluents include isotonic saline solutions, for example phosphate-buffered saline. Suitable diluents and excipients also include, for example, water, saline, dextrose, glycerol, or the like, and combinations thereof. In addition, if desired substances such as wetting or emulsifying agents, stabilizing or ph buffering agents may also be present.

The term "pharmaceutically acceptable carrier" refers to any pharmaceutical carrier that does not itself induce the production of antibodies harmful to the individual receiving the composition, and which can be administered without undue toxicity. Suitable carriers can be large, slowly metabolized macromolecules such as proteins, polysaccharides, polylactic acids, polyglycolic acids, polymeric amino acids, and amino acid copolymers.

Pharmaceutically acceptable salts can also be present, *e.g.,* mineral acid salts such as hydrochlorides, hydrobromides, phosphates, sulfates, and the like; and the salts of organic acids such as acetates, propionates, malonates, benzoates, and the like.

Suitable carrier materials include any carrier or vehicle commonly used as a base for creams, lotions, sprays, foams, gels, emulsions, lotions or paints for topical administration. Examples include emulsifying agents, inert carriers including hydrocarbon bases, emulsifying bases, non-toxic solvents or water-soluble bases. Particularly suitable examples include pluronics, HPMC, CMC and other cellulose-based ingredients, lanolin, hard paraffin, liquid paraffin, soft yellow paraffin or soft white paraffin, white beeswax, yellow beeswax, cetostearyl alcohol, cetyl alcohol, dimethicones, emulsifying waxes, isopropyl myristate, microcrystalline wax, oleyl alcohol and stearyl alcohol.

Preferably, the pharmaceutically acceptable carrier or vehicle is a gel, suitably a nonionic polyoxyethylene-polyoxypropylene copolymer gel, for example, a Pluronic gel, preferably Pluronic F-127 (BASF Corp.). This gel is particularly preferred as it is a liquid at low temperatures but rapidly sets at physiological temperatures, which confines the release of the ODN component to the site of application or immediately adjacent that site.

An auxiliary agent such as casein, gelatin, albumin, glue, sodium alginate, carboxymethylcellulose, methylcellulose, hydroxyethylcellulose or polyvinyl alcohol may also be included in the formulation of the invention.

Other suitable formulations include pluronic gel-based formulations, carboxymethylcellulose(CMC)-based formulations, and hyroxypropylmethylcellulose(HPMC)-based formulations. The composition may be formulated for any desired form of delivery, including topical, instillation, parenteral, intramuscular, subcutaneous, or transdermal administration. Other useful formulations include slow or delayed release preparations.

Where the anti-connexin agent is a nucleic acid, such as a polynucleotide, uptake of nucleic acids by mammalian cells is enhanced by several known transfection techniques for example those including the use of transfection agents. Such techniques may be used with certain anti-connexin agents, including polynucleotides. The formulation which is administered may contain such transfection agents. Examples of these agents include cationic agents (for example calcium phosphate and DEAE-dextran) and lipofectants (for example lipofectam™ and transfectam™)), and surfactants.

Where the anti-connexin agent comprises a polynucleotide, conveniently, the formulation further includes a surfactant to assist with polynucleotide cell penetration or the formulation may contain any suitable loading agent. Any suitable non-toxic surfactant may be included, such as DMSO. Alternatively a transdermal penetration agent such as urea may be included.

The effective dose for a given subject or condition can be determined by routine experimentation or other methods known in the art or later developed. For example, in order to formulate a range of dosage values, cell culture assays and animal studies can be used. The dosage of such compounds preferably lies within the dose that is therapeutically effective for at least 50% of the population, and that exhibits little or no toxicity at this level.

The effective dosage of each of the anti-connexin agents employed in the methods and compositions of the invention may vary depending on a number of factors including the particular anti-connexin agent or agents employed, the mode of administration, the frequency of administration, the condition being treated, the severity of the condition being treated, the route of administration, the needs of a patient sub-population to be treated or the needs of the individual patient which different needs can be due to age, sex, body weight, general condition of the patient, relevant medical condition specific to the patient.

A suitable dose may be based on the amount of anti-connexin agent per kg of body weight of the subject, and include, from about 0.001 to about 1 mg/kg body weight such as about 0.01 to about 0.4 mg/kg body weight. A suitable dose may however be from about 0.001 to about 0.1 mg/kg body weight such as about 0.01 to about 0.050 mg/kg body weight. Doses from about 1 to 100, about 100-200, about 200-300, about 300-400, and about 400-500 micrograms or more, and up to about 750-1000 micrograms are appropriate for certain indications.

The dose at which an anti-connexin agent is administered to a patient will depend upon a variety of factors such as the age, weight and general condition of the patient, the condition that is being treated, and the particular anti-connexin agent that is being administered.

A suitable therapeutically effective dose of an anti-connexin agent may be from about 0.001 to about 1 mg/kg body weight such as about 0.01 to about 0.4 mg/kg body weight. A suitable dose may however be from about 0.001 to about 0.1 mg/kg body weight such as about 0.01 to about 0.050 mg/kg body weight.

Therapeutically effective doses of anti-connexin agents from about 1 to 100, 100-200, 100- or 200-300, 100- or 200- or 300-400, and 100- or 200- or 300- or 400-500 micrograms are appropriate. Doses from about 1-1000 micrograms are also appropriate. Doses up to 2 milligrams may also be used. Doses are adjusted appropriately when the anti-connexin agent or agents are provided in the form of a dressing, typically upward to maintain the desired total dose administration.

Alternatively, in the case of anti-connexin oligonucleotides or anti-connexin peptidomimetics, the dosage of each of the gap junction modulation agents in the compositions may be determined by reference to the composition's concentration relative to the size, length, depth, area or volume of the area to which it will be applied. For example, in certain topical applications, dosing of the pharmaceutical compositions may be calculated based on mass (*e.g.* grams) of or the concentration in a pharmaceutical composition (*e.g.* µg/ul) per length, depth, area, or volume of the area of application. Useful doses range from about 1 to about 10 micrograms per square centimeter of wound size. Certain doses will be about 1-2, about 1-5, about 2-4, about 5-7, and about 8-10 micrograms per square centimeter of wound size. Other useful doses are greater than about 10 micrograms per square centimeter of wound size, including at least about 15 micrograms per square centimeter of wound size, at least about 20 micrograms per square centimeter of wound size, at least about 25 micrograms per square centimeter of wound size, about 30 micrograms per square centimeter of wound size, at least about 35 micrograms per square centimeter of wound size, at least about 40 micrograms per square centimeter of wound size, at least about 50 micrograms per square centimeter of wound size, and at least about 100 to at least about 150 micrograms per square centimeter of wound size. Other doses include about 150-200 micrograms per square centimeter, about 200-250 micrograms per square centimeter, about 250-300 micrograms per square centimeter, about 300-350 micrograms per square centimeter, about 350-400 micrograms per square centimeter, and about 400-500 micrograms per square centimeter.

In certain embodiments, the anti-connexin agent composition may be applied at about 0.01 micromolar (µM) or 0.05 µM to about 200 µM, or up to 300 µM or up to 1000 µM or up to 2000 µM or up to 3200 µM or more final concentration at the treatment site and/or adjacent to the treatment site, and any doses and dose ranges within these dose numbers. Preferably, the antisense polynucleotide composition is applied at about 0.05 µM to about 100 µM final concentration, more preferably, the anti-connexin agent composition is applied at about 1.0 µM to about 50 µM final concentration, and more preferably, the anti-connexin agent composition is applied at about 5-10 µM to about 30-50 µM final concentration. Additionally, the combined anti-connexin agent composition is applied at about 8 µM to about 20 µM final concentration, and alternatively the anti-connexin agent composition is applied at about 10 µM to about 20 µM final concentration, or at about 10 to about 15 µM final concentration. In certain other embodiments, the anti-connexin agent is applied at about 10 µM final concentration. In yet another embodiment, the anti-connexin agent composition is applied at about 1-15 µM final concentration. In other embodiements, the anti-connexin agent is applied at about a 20 µM, 30 µM, 40 µM, 50 µM, 60 µM, 70 µM, 80 µM, 90 µM, 100 µM., 10-200 µM, 200-300 µM, 300-400 µM, 400-500 µM, 500-600 µM, 600-700 µM, 700-800 µM, 800-900 µM, 900-1000 or 1000-1500 µM , or 1500 µM - 2000 µM or 2000 µM - 3000 µM or greater.

Anti-connexin agent dose amounts include, for example, about 0.1-1, 1-2, 2-3, 3-4, or 4-5 micrograms (µg), from about 5 to about 10 µg, from about 10 to about 15 µg, from about 15 to about 20 µg, from about 20 to about 30 µg, from about 30 to about 40 µg, from about 40 to about 50 µg, from about 50 to about 75 µg, from about 75 to about 100 µg, from about 100 µg to about 250 µg, and from 250 µg to about 500 µg. Dose amounts from 0.5 to about 1.0 milligrams or more or also provided, as noted above. Dose volumes will depend on the size of the site to be treated, and may range, for example, from about 25-100 µL to about 100-200 µL, from about 200-500 µL to about 500-1000 µL. Milliliter doses are also appropriate for larger treatment sites.

Still other dosage levels between about 1 nanogram (ng)/kg and about 1 mg/kg body weight per day of each of the agents described herein. In certain embodiments, the dosage of each of the subject compounds will generally be in the range of about 1 ng to about 1 microgram per kg body weight, about 1 ng to about 0.1 microgram per kg body weight, about 1 ng to about 10 ng per kg body weight, about 10 ng to about 0.1 microgram per kg body weight, about 0.1 microgram to about 1 microgram per kg body weight, about 20 ng to about 100 ng per kg body weight, about 0.001 mg to about 0.01 mg per kg body weight, about 0.01 mg to about 0.1 mg per kg body weight, or about 0.1 mg to about 1 mg per kg body weight. In certain embodiments, the dosage of each of the subject compounds will generally be in the range of about 0.001 mg to about 0.01 mg per kg body weight, about 0.01 mg to about 0.1 mg per kg body weight, about 0.1 mg to about 1 mg per kg body weight. If more than one anti-connexin agent is used, the dosage of each anti-connexin agent need not be in the same range as the other. For example, the dosage of one anti-connexin agent may be between about 0.01 mg to about 10 mg per kg body weight, and the dosage of another anti-connexin agent may be between about 0.1 mg to about 1 mg per kg body weight.

In certain embodiments, the anti-connexin agent composition may be applied at about 0.01 micromolar (µM) or 0.05 µM to about 200 µM final concentration at the treatment site and/or adjacent to the treatment site. Preferably, the antisense polynucleotide composition is applied at about 0.05 µM to about 100 µM final concentration, more preferably, the anti-connexin agent composition is applied at about 1.0 µM to about 50 µM final concentration, and more preferably, the anti-connexin agent composition is applied at about 5-10 µM to about 30-50 µM final concentration. Additionally, the combined anti-connexin agent composition is applied at about 8 µM to about 20 µM final concentration, and alternatively the anti-connexin agent composition is applied at about 10 µM to about 20 µM final concentration, or at about 10 to about 15 µM final concentration. In certain other embodiments, the anti-connexin agent is applied at about 10 µM final concentration. In yet another embodiment, the anti-connexin agent composition is applied at about 1-15 µM final concentration. Anti-connexin agent dose amounts include, for example, about 0.1-1, 1-2, 2-3, 3-4, or 4-5 micrograms (µg), from about 5 to about 10 µg, from about 10 to about 15 µg, from about 15 to about 20 µg, from about 20 to about 30 µg, from about 30 to about 40 µg, from about 40 to about 50 µg, from about 50 to about 75 µg, from about 75 to about 100 µg, from about 100 µg to about 250 µg, and from 250 µg to about 500 µg. Dose amounts from 0.5 to about 1.0 milligrams or more or also provided, as noted above. Dose volumes will depend on the size of the site to be treated, and may range, for example, from about 25-100 µL to about 100-200 µL, from about 200-500 µL to about 500-1000 µL. Milliliter doses are also appropriate for larger treatment sites.

Still other dosage levels between about 1 nanogram (ng)/kg and about 1 mg/kg body weight per day of each of the agents described herein. In certain embodiments, the dosage of each of the subject compounds will generally be in the range of about 1 ng to about 1 microgram per kg body weight, about 1 ng to about 0.1 microgram per kg body weight, about 1 ng to about 10 ng per kg body eight, about 10 ng to about 0.1 microgram per kg wody weight, about 0.1 microgram to about 1 microgram per kg body weight, about 20 ng to about 100 ng per kg bodyweight, about 0.001 mg to about 001 mg per kg body weight, about 0.01 mg to about 0.1 mg per kg body weight, or about 0.1 mg to about 1 mg per kg body weight. In certain embodiments, the dosage of each of the subject compounds will generally be in the range of about 0.001 mg to about 0.01 mg per kg body weight, about 0.01 mg to about 0.1 mg per kg body weight, about 0.1 mg to about .1 mg per kg body weight. If more than one anti-connexin agent is used, the dosage of each anti-connexin agent need not be in the same range as the other. For example, the dosage of one anti-connexion agent may be between 0.01 mg to about 10 mg per kg body weight, and the dosage of anotehr anti-connexion agent may be between about 0.1 mg to about 1 mg per kg bodyweight.

Conveniently, the composition is administered in a sufficient amount to downregulate expression of said connexin proteins, or modulate gap junction formation or connexin opening for at least about 0.5 to 1 hour, at least about 1-2 hours, at least about 2-4 hours, at least about 4-6 hours, at least about 6-8 hours, at least about 8-10 hours, at least about 12 hours, or at least about 24 hours post-administration.

The dosage of each of the anti-connexin agents in the compositions and methods of the subject invention may also be determined by reference to the concentration of the composition relative to the size, length, depth, area or volume of the area to which it will be applied. For example, in certain topical and other applications, *e*.*g*., instillation, dosing of the pharmaceutical compositions may be calculated based on mass (*e.g.* micrograms) of or the concentration in a pharmaceutical composition (*e.g.* µg/ul) per length, depth, area, or volume of the area of application.

Thus, in the case of anti-connexin oligonucleotides or anti-connexin peptidomimetics, the dosage of each of the gap junction modulating agents in the compositions may be determined by reference to the composition's concentration relative to the size, length, depth, area or volume of the area to which it will be applied. For example, in certain topical applications, dosing of the pharmaceutical compositions may be calculated based on mass (*e.g.* grams) of or the concentration in a pharmaceutical composition (*e.g.* ug/ul) per length, depth, area, or volume of the area of application. Useful doses range from about 1 to about 10 micrograms per square centimeter of wound size. Certain doses will be about 1-2, about 1-5, about 2-4, about 5-7, and about 8-10 micrograms per square centimeter of wound size. Other useful doses are greater than about 10 micrograms per square centimeter of wound size, including at least about 15 micrograms per square centimeter of wound size, at least about 20 micrograms per square centimeter of wound size, at least about 25 micrograms per square centimeter of wound size, about 30 micrograms per square centimeter of wound size, at least about 35 micrograms per square centimeter of wound size, at least about 40 micrograms per square centimeter of wound size, at least about 50 micrograms per square centimeter of wound size, and at least about 100 to at least about 150 micrograms per square centimeter of wound size. Other doses include about 150-200 micrograms per square centimeter, about 200-250 micrograms per square centimeter, about 250-300 micrograms per square centimeter, about 300-350 micrograms per square centimeter, about 350-400 micrograms per square centimeter, and about 400-500 micrograms per square centimeter.

All doses and dose ranges referenced herein are applicable, for example, to anti-connexin oligonucleotides. These dose ranges are also applicable, for example, to anti-connexin peptides anti-connexin mimetic peptides and anti-connexin peptidomimetics.

Conveniently, the anti-connexin agent is administered in a sufficient amount to downregulate expression of a connexin protein, or modulate gap junction formation or connexin opening for at least about 0.5 to 1 hour, at least about 1-2 hours, at least about 2-4 hours, at least about 4-6 hours, at least about 6-8 hours, at least about 8-10 hours, at least about 12 hours, or at least about 24 hours post-administration.

The dosage of each of the anti-connexin agents in the compositions and methods of the subject invention may also be determined by reference to the concentration of the composition relative to the size, length, depth, area or volume of the area to which it will be applied. For example, in certain topical and other applications, *e.g.,* instillation, dosing of the pharmaceutical compositions may be calculated based on mass (*e.g.* micrograms) of or the concentration in a pharmaceutical composition (*e.g.* µg/µl) per length, depth, area, or volume of the area of application.

As noted herein, the doses of an anti-connexin polynucleotide, peptide or peptidomimetic administered in combination, or other anti-connexin agents administered in combination with either or both, can be adjusted down from the doses administered when given alone.

The doses may be administered in single or divided applications. The doses may be administered once, or application may be repeated. Typically, application will be repeated weekly until wound healing is promoted, or a repeat application may be made in the event that wound healing slows or is stalled. Doses may be applied 3-7 days apart, or more. In the case of a chronic wound, repeat applications may be made, for example, weekly, or biweekly, or monthly or in other frequency for example if and when wound healing slows or is stalled. For some indications, such as certain ocular uses, more frequent dosing, up to hourly may employed.

Administration of anti-connexin agent(s), e.g., for downregulation of connexin expression, or blockade or inhibition of connexin opening or activity, therefore will modulate communication between the cells, or loss into the extracellular space in the case of connexin regulation, and minimize additional cell loss or injury or consequences of injury.

While the delivery period will be dependent upon both the site at which the downregulation is to be induced and the therapeutic effect which is desired, continuous or slow-release delivery for about 0.5-1 hour, about 1-2 hours, about 2-4 hours, about 4-6 hours, about 6-8, or about 24 hours or longer is provided. In accordance with the present invention, this is achieved by inclusion of one or more anti-connexin agents in a formulation together with a pharmaceutically acceptable carrier or vehicle, particularly in the form of a formulation for continuous or slow-release administration.

As noted, the one or more agents of the invention may be administered before, during, immediately following wounding, for example, or within about 180, about 120, about 90, about 60, or about 30 days, but preferably within about 10, about 9, about 8, about 7, about 6, about 5, about 4, about 3, or about 2 days or less, and most preferably within about 24, about 12, about 10, about 9, about 8, about 7, about 6, about 5, about 4, about 3, about 2 hours or within about 60, about 45, about 30, about 15, about 10, about 5, about 4, about 3, about 2, about 1 minute following wounding, for example.

The routes of administration and dosages described herein are intended only as a guide since a skilled physician will determine the optimum route of administration and dosage for any particular patient and condition.

Any of the methods of treating a subject having a wound and/or condition referenced or described herein may utilize the administration of any of the doses, dosage forms, formulations, and/or compositions herein described.

Agents useful for wound healing suitable for the preparation of the pharmaceutical compositions described herein may be prepared and administered using methods as known in the art (see, for example, U.S. Patent Nos. 7,098,190, 6,319,907, 6,331,298, 6,387,364, 6,455,569, 6,566,339, 6,696,433, 6,855,505, 6,900,181, 7,052,684 and EP1100529 B1. The concentration of each anti-connexin polynucleotide and agents useful for wound healing need not be in the same range as the other. Other amounts will be known to those of skill in the art and readily determined. For example, suitable combination dosages and formulations in accordance with various aspects and embodiments as described herein may be administered according to the dosing regimen as described in US6903078 to Lewis entitled "Combination PDGF, KGF, IGF, and IGFBP for wound healing."

The initial and any subsequent dosages administered will depend upon the patient's age, weight, condition, and the disease, wound, disorder or biological condition being treated. Depending on the agent usedul for wound healing, the dosage and protocol for administration will vary, and the dosage will also depend on the method of administration selected, for example, local or systemic administration.

The agent useful for wound healing may be applied internally or externally, and may be directed towards any tissue exhibiting a wound. For topical administration of IGF, for example, a zinc oxide formulation can be applied, which induces the local production of IGF, as described in Tarnow et al, Scand J. Plast Reconstr Hand Surg. 28: 255-259 (1994). An effective dose of PDGF has been reported to be 5 ng/mm² or higher when applied topically as described in U.S. Pat. No. 4,861,757, and at least 1 ng/ml local concentration of an isoform of PDGF (for example, PDGF-AA, PDGF-BB, or PDGF-AB), up to about 30 ng/ml local concentration applied to a population of fibroblasts as described in Lepisto et al., Biochem Biophys Res. Comm 209: 393-399 (1995). PDGF can be administered in a carboxymethylcellulose gel formulation at concentrations of about 10 µg/gm to about 500 µg/gm of gel, about 20 µg/gm to about 200 µg/gm, and about 30 µg/gm to about 100 µ g/gm of gel, optimally about 100 µg/gm of gel. Efficacy of PDGF has been achieved within the range of about 3 µg/ml solution to about 300 µg/ml of solution administered.

About 50 µl of KGF of a concentration of about 5 µg/ml may be effective for wound healing by topical application to epithelial tissue as described in Sotozono et al, Invest. Opthal. Vis. Science 36: 1524-29 (1995). As described in U.S. Pat. No. 4,861,757, an effective amount of IGF when co-administered with PDGF is in the range of at least 2.5 ng/mm² to about 5 ng/mm², with a ratio of PDGF to IGF in the range of about 1:10 to about 25:1 weight to weight, with the most effective ratios being PDGF to IGF of about 1:1 to about 2:1 weight to weight. IGFBP administered in combination with IGF has been shown to increase wound healing at dose levels of about 5 µg of IGF with about 1.5 µg of phosphorylated IGFBP in a molar ration of about 11:1 IGF:IGFBP, as described in Jyung et al, Surgery 115:233-239 (1994).

For administration of polypeptide therapeutics, for example, PDGF, KGF, IGF and IGFBP polypeptides, the dosage can be in the range of about 5 µg to about 50 µg/kg of tissue to which the application is directed, also about 50 µg to about 5 mg/kg, also about 100 µg to about 500 µg/kg of tissue, and about 200 to about 250 µg/kg. For polynucleotide therapeutics, for example in a gene therapy administration protocol, depending on the expression strength the polynucleotide in the patient, for tissue targeted administration, vectors containing expressible constructs including PDGF, KGF, IGF, and IGFBP coding sequences can be administered in a range of about 100 ng to about 200 mg of DNA for local administration in a gene therapy protocol, also about 500 ng to about 50 mg, also about 1 µg to about 2 mg of DNA, about 5 µg of DNA to about 500 µg of DNA, and about 20 µg to about 100 µg during a local administration in a gene therapy protocol, and about 250 µg, per injection or administration. Factors such as method of action and efficacy of transformation and expression are therefore considerations that will effect the dosage required for ultimate efficacy for administration of DNA therapeutics. Where greater expression is desired, over a larger area of tissue, larger amounts of DNA or the same amounts re-administered in a successive protocol of administrations, or several administrations to different adjacent or close tissue portions of for example, a wound site may be required to effect a positive therapeutic outcome.

Therapeutic agents and anti-microtubule agents suitable for the preparation of the pharmaceutical compositions described herein may be formulated and administered using methods as known in the art. The initial and any subsequent dosages administered will depend upon the patient's age, weight, condition, and the disease, wound, disorder or biological condition being treated. Depending on the therapeutic, the dosage and protocol for administration will vary, and the dosage will also depend on the method of administration selected, for example, local or systemic administration.

As noted herein, the doses of either an anti-connexin polynucleotides or another agent administered in combination can be adjusted down from the doses administered when given alone.

The combined use of several agents may reduce the required dosage for any individual agent because the onset and duration of effect of the different agents may be complementary. In a preferred embodiment, the combined use of one or more anti-connexin polynucleotides and one or more therapeutic agents, agents useful for wound healing, and/or anti-microtubule agents has an additive, synergistic or super-additive effect.

In some cases, the combination of one or more anti-connexin polynucleotides and one or more therapeutic agents, one or more agents useful for wound healing, and/or one or more anti-microtubule agents have an additive effect. In other cases, the combination can have greater-than-additive effect. Such an effect is referred to herein as a "supra-additive" effect, and may be due to synergistic or potentiated interaction.

The term "supra-additive promotion of wound healing" refers to a mean wound healing produced by administration of a combination of an anti-connexin polynucleotide and one or more therapeutic agents, agents useful for wound healing and/or anti-microtubule agents, is statistically significantly higher than the sum of the wound healing produced by the individual administration of either any of the agents alone. Whether produced by combination administration of an anti-connexin polynucleotide and one or more therapeutic agents, agents useful for wound healing, and/or anti-microtubule agents is "statistically significantly higher" than the expected additive value of the individual compounds may be determined by a variety of statistical methods as described herein and/or known by one of ordinary skill in the art. The term "synergistic" refers to a type of supra-additive inhibition in which both the anti-connexin polynucleotide and one or more therapeutic agents, agents useful for wound healing and/or anti-microtubule agents individually have the ability to promote wound healing or reduce fibrosis and scarring. The term "potentiated" refers to type of supra-additive effect in which one of the anti-connexin polynucleotide or one or more therapeutic agents, agents useful for wound healing, and/or anti-microtubule agents individually has the increased ability to promote wound healing.

In general, potentiation may be assessed by determining whether the combination treatment produces a mean wound healing increase in a treatment group that is statistically significantly supra-additive when compared to the sum of the mean wound healing increases produced by the individual treatments in their treatment groups respectively. The mean wound healing increase may be calculated as the difference between control group and treatment group mean wound healing. The fractional increase in wound healing, "fraction affected" (Fa), may be calculated by dividing the treatment group mean wound healing increase by control group mean wound healing. Testing for statistically significant potentiation requires the calculation of Fa for each treatment group. The expected additive Fa for a combination treatment may be taken to be the sum of mean Fas from groups receiving either element of the combination. The Two-Tailed One-Sample T-Test, for example, may be used to evaluate how likely it is that the result obtained by the experiment is due to chance alone, as measured by thep-value. Ap-value of less than.05 is considered statistically significant, that is, not likely to be due to chance alone. Thus, Fa for the combination treatment group must be statistically significantly higher than the expected additive Fa for the single element treatment groups to deem the combination as resulting in a potentiated supra-additive effect.

Whether a synergistic effect results from a combination treatment may be evaluated by the median-effect/combination-index isobologram method (Chou, T., and Talalay, P. (1984) Ad. Enzyme Reg. 22:27-55). In this method, combination index (CI) values are calculated for different dose-effect levels based on parameters derived from median-effect plots of the anti-connexin polynucleotide alone, the one or more agents useful for wound healing alone, and the combination of the two at fixed molar ratios. CI values of & It; 1 indicate synergy, CI-1 indicates an additive effect, and CP1 indicates an antagonistic effect. This analysis may be performed using computer software tools, such as CalcuSyn, Windows Software for Dose Effect Analysis (Biosoft(D, Cambridge UK).

Any method known or later developed in the art for analyzing whether a supra-additive effect exists for a combination therapy is contemplated for use in screening for suitable anti-connexin polynucleotides for use in combination with one or more therapeutic agents, agents useful for wound healing and/or anti-microtubule agents.

In another preferred embodiment, the combined use of one or more anti-connexin polynucleotides and one or more therapeutic agents, agents useful for wound healing, and/or anti-microtubule agents reduces the effective dose of any such agent compared to the effective dose when said agent administered alone. In certain embodiments, the effective dose of the agent when used in combination with one or more anti-connexin polynucleotides is about 1/15 to about 1/2, about 1/10 to about 1/3, about 1/8 to about 1/6, about 1/5, about 1 /4, about 1/3 or about 1/2 the dose of the agent when used alone.

In another preferred embodiment, the combined use of one or more anti-connexin polynucleotides and one or more therapeutic agents, agents useful for wound healing, and/or anti-microtubule agents reduces the frequency in which said agent is administered compared to the frequency when said agent is administered alone. Thus, these combinations allow the use of lower and/or fewer doses of each agent than previously required to achieve desired therapeutic goals.

The doses may be administered in single or divided applications. The doses may be administered once, or application may be repeated. Repeat applications are typically applied about once per week, or when wound-healing may appear to be stalled or slowing.

The anti-connexin agent (with or without one ore more therapeutic agents, agents useful for wound healing and/or anti-microtubule agetns) can be administered in any manner which achieves a desired result. Preferred methods include peritubular administration (either direct application at the time of surgery or with endoscopic, ultrasound, CT, MRI, or fluoroscopic guidance); "coating" the surgical implant; and placement of a drug-eluting polymeric implant at the surgical site. In a preferred embodiment, 0.5% to 20% anti-connexin polynucleotide by weight is loaded into a polymeric carrier (as described in the following examples) and applied to the peritubular (mesenteric) surface as a "paste", "film", or "wrap" which releases the drug over a period of time. During endoscopic procedures, the anti-connexin polymer preparation may be applied as a "spray", via delivery ports in the endoscope, to the mesentery of the abdominal and pelvic organs manipulated during the operation. In a particularly preferred embodiment, the peritubular composition is about 0.1% to about 5% anti-connexin polynucleotide by weight. In another preferred embodiment, a polymeric coating containing about 0.1% to about 20% or more or an anti-connexin agent is applied to the surface of the surgical implant (e.g., breast implant, artificial joint, vascular graft, *etc.*). In yet another preferred embodiment, a polymeric implant containing about 0.01% to about 20% or more of an anti-connexin agent by weight is applied directly to the surgical site (*e.g.,* directly into the sinus cavity, chest cavity, abdominal cavity, or at the operative site during neurosurgery).

In another embodiment, lavage fluid containing about 1 to about 100 µg/cm² (preferably about 10 to about 50 µg /cm²) of an anti-connexin agent, would be used at the time of or immediately following surgery and administered during surgery or intraperitoncally, by a physician. In all of the embodiments, other anti-connexin polynucleotides would be administered at equivalent doses adjusted for potency and tolerability of the polynucleotide.

The anti-connexin agents of the invention may be administered by the same or different routes. Preferably an anti-connexin agent is delivered by topical administration (peripherally or directly to a site), including but not limited to topical administration using solid supports (such as dressings and other matrices) and medicinal formulations (such as gels, mixtures, suspensions and ointments). In one embodiment, the solid support comprises a biocompatible membrane or insertion into a treatment site. In another embodiment, the solid support comprises a dressing or matrix. In one embodiment of the invention, the solid support composition may be a slow release solid support composition, in which the anti-connexin agent is dispersed in a slow release solid matrix such as a matrix of alginate, collagen, or a synthetic bioabsorbable polymer. Preferably, the solid support composition is sterile or low bio-burden. In one embodiment, a wash solution comprising an anti-connexin agent can be used.

The anti-connexin agents may be administered topically or by instillation, for example (at or near the site to be treated). Preferably the anti-connexin agents are combined with a pharmaceutically acceptable carrier or diluent to produce a pharmaceutical composition. Suitable carriers and diluents include isotonic saline solutions, for example phosphate-buffered saline.

The therapeutic agent may be administered by intra-articular, periarticular, peritendinal or soft tissue injection. The therapeutic agent may be injected as a single dose or in multiple doses. It is contemplated that the methods and composition described herein may be administration using various methods known in the art or later developed, including, for example, laproscopic techniques. Exemplary techniques may include intraoperative techniques and/or direct injection, such as, for example, aspiration prior to injection; stepwise injection into the insertions and origins of the medial and lateral collateral ligaments, the synovium, and the cut edge of the quadriceps tendon; hip injections into anterior capsule and the insertion of the gluteus medius, the posterior capsule and short external rotators, the gluteus medius, and the cut edges of the fascia lata. In addition, it is contemplated that the compositions described herein may be injected either directly or intra-operatively into articular capsules or bone recesses to produce a "depot" effect. Such depot effect may facilitate drug delivery in a time-release manner leading to improved bioavailability and/or distribution which enhances therapeutic efficacy.

After the traumatic event, a needle (using sterile technique) may be used to introduce the therapeutic compound intra-articularly. In the case of an elbow, a 25G needle is introduced between the radial head and olecranon process. Range of motion is commenced immediately after injection if permissible or in the case of a fracture is treated as per standard protocol. In the case of a shoulder, a posterior or posterior-lateral approach can be used with a 20G to 25G 1.5 inch needle. In the case of a knee, the knee can be approached antero-medially, antro-laterally or via supro-lateral approach with the same size needle to introduce the anti-connexin agent. Other intra-articular injection methods are well known in the art. The routes of administration and dosages described above are intended only as a guide since a skilled physician will be able to determine readily the optimum route of administration and dosage for any particular patient and condition.

As noted, the anti-connexin agent may be administered before, during, immediately following surgery or wounding, for example, or within about 180, about 120, about 90, about 60, or about 30 days, but preferably within about 10, about 9, about 8, about 7, about 6, about 5, about 4, about 3, or about 2 days or less, and most preferably within about 24, about 12, about 10, about 9, about 8, about 7, about 6, about 5, about 4, about 3, about 2 hours or within about 60, about 45, about 30, about 15, about 10, about 5, about 4, about 3, about 2, about 1 minute following a surgery or other orthopedic procedure leading to tissue damage, for example.

Conveniently, the composition is administered in a sufficient amount to downregulate expression of said connexin protein(s) for at least about 1-2 hours, at least about 2-4 hours, at least about 4-6 hours, at least about 6-8 hours, at least about 8-10 hours, at least about 12 hours, or at least about 24 hours post-administration.

The routes of administration and dosages described herein are intended only as a guide since a skilled physician will be able to determine readily the optimum route of administration and dosage for any particular patient and condition.

Optionally, the anti-connexin agent may be formulated with one or more therapeutic agents, agents useful for wound healing, and/or anti-microtubule agents. The inclusion of one or more such agents may allow a synergistic effect on the prevention or treatment of chronic wounds. Such additional agent(s) may be administered separately, simultaneously or sequentially with the anti-connexin agent. Therapeutic agents include, for example, anti-infectives, anaesthetics, analgesics, antibiotics, narcotics, and steroidal and non-steroidal anti-inflammatory agents. In certain embodiments, one, two three, four, five or six therapeutic agents may be used in combination.

Any of the methods of treating a subject having or suspected of having or predisposed to, or at risk for, a disease, disorder, and/or condition, referenced or described herein may utilize the administration of any of the doses, dosage forms, formulations, and/or compositions herein described.

### Dressings and Matrices

In one aspect, the anti-connexin agent, alone or in combination with one or more therapeutic agents, agents useful for wound healing, and/or anti-microtubule agents are provided in the form of a dressing or matrix. In certain embodiments, the one or more agents of the invention are provided in the form of a liquid, semi solid or solid composition for application directly, or the composition is applied to the surface of, or incorporated into, a solid contacting layer such as a dressing gauze or matrix. The dressing composition may be provided for example, in the form of a fluid or a gel. The anti-connexin agent alone or in combination with one or more therapeutic agents, agents useful for wound healing, and/or anti-microtubule agents may be provided in combination with conventional pharmaceutical excipients for topical application. Suitable carriers include: Pluronic gels, Polaxamer gels, Hydrogels containing cellulose derivatives, including hydroxyethyl cellulose, hydroxymethyl cellulose, carboxymethyl cellulose, hydroxypropylmethyl cellulose and mixtures thereof; and hydrogels containing polyacrylic acid (Carbopols). Suitable carriers also include creams/ointments used for topical pharmaceutical preparations, e.g., creams based on cetomacrogol emulsifying ointment. The above carriers may include alginate (as a thickener or stimulant), preservatives such as benzyl alcohol, buffers to control pH such as disodium hydrogen phosphate/sodium dihydrogen phosphate, agents to adjust osmolarity such as sodium chloride, and stabilizers such as EDTA.

In addition to the biological matrices previously mentioned, suitable dressings or matrices may include, for example, the following with one or more anti-connexin polynucleotides and one or more anti-connexin peptides or peptidomimetics (or other anti-connexin agents to be administered in combination with either or both):
1) Absorptives: suitable absorptives may include, for example, absorptive dressings, which can provide, for example, a semi-adherent quality or a non-adherent layer, combined with highly absorptive layers of fibers, such as for example, cellulose, cotton or rayon. Alternatively, absorptives may be used as a primary or secondary dressing.
2) Alginates: suitable alginates include, for example, dressings that are non-woven, non-adhesive pads and ribbons composed of natural polysaccharide fibers or xerogel derived from seaweed. Suitable alginates dressings may, for example, form a moist gel through a process of ion exchange upon contact with exudate. In certain embodiments, alginate dressings are designed to be soft and conformable, easy to pack, tuck or apply over irregular-shaped areas. In certain embodiments, alginate dressings may be used with a second dressing.
3) Antimicrobial Dressings: suitable antimicrobial dressings may include, for example, dressings that can facilitate delivery of bioactive agents, such as, for example, silver and polyhexamethylene biguanide (PHMB), to maintain efficacy against infection, where this is needed or desirable. In certain embodiments, suitable antimicrobial dressings may be available as for example, as sponges, impregnated woven gauzes, film dressings, absorptive products, island dressings, nylon fabric, non-adherent barriers, or a combination of materials.
4) Biological & Biosynthetics: suitable biological dressings or biosynthetic dressings may include, for example, gels, solutions or semi-permeable sheets derived from a natural source, *e.g.,* pigs or cows. In certain embodiments, a gel or solution is applied to the treatment site and covered with a dressing for barrier protection. In another embodiment, a biological-based (e.g., pig intestinal mucosa or bladder tissue) or biosynthetic-based sheet is placed *in situ* which may act as membrane, remaining in place after a single application, or the may be biological dressings or biosynthetic dressings may be prepared in advance to include one or more, preferably two, anti-connexin agents.
5) Collagens: suitable collagen dressings may include, for example, gels, pads, particles, pastes, powders, sheets or solutions derived from for example, bovine, porcine or avian sources or other natural sources or donors. In certain embodiments, the collagen dressing may interact with treatment site exudate to form a gel. In certain embodiments, collagen dressing may be used in combination with a secondary dressing.
6) Composites: suitable composite dressings may include, for example, dressings that combine physically distinct components into a single product to provide multiple functions, such as, for example, a bacterial barrier, absorption and adhesion. In certain embodiment, the composite dressings are comprised of, for example, multiple layers and incorporate a semi-or non-adherent pad. In certain embodiment, the composite may also include for example, an adhesive border of non-woven fabric tape or transparent film. In certain other embodiment, the composite dressing may function as for example, either a primary or a secondary dressing and in yet another embodiment, the dressing may be used in combination with topical pharmaceutical composition.
7) Contact Layers: suitable contact layer dressings may include, for example, thin, non-adherent sheets placed on an area to protect tissue from for example, direct contact with other agents or dressings applied to the treatment site. In certain embodiments, contact layers may be deployed to conform to the shape of the area of the treatment site and are porous to allow exudate to pass through for absorption by an overlying, secondary dressing. In yet another embodiment, the contact layer dressing may be used in combination with topical pharmaceutical composition.
8) Elastic Bandages: suitable elastic bandages may include, for example, dressings that stretch and conform to the body contours. In certain embodiment, the fabric composition may include for example, cotton, polyester, rayon or nylon. In certain other embodiments, the elastic bandage may for example, provide absorption as a second layer or dressing, to hold a cover in place, to apply pressure or to cushion a treatment site.
9) Foams: suitable foam dressings may include, for example, sheets and other shapes of foamed polymer solutions (including polyurethane) with small, open cells capable of holding fluids. Exemplary foams may be for example, impregnated or layered in combination with other materials. In certain embodiment, the absorption capability may be adjusted based on the thickness and composition of the foam. In certain other embodiments, the area in contact with the treatment site may be non-adhesive for easy removal. In yet another embodiment, the foam may be used in combination with an adhesive border and/or a transparent film coating that can serve as an anti-infective barrier.
10) Gauzes & Non-Woven dressings: suitable gauze dressings and woven dressings may include, for example, dry woven or non-woven sponges and wraps with varying degrees of absorbency. Exemplary fabric composition may include, for example, cotton, polyester or rayon. In certain embodiment, gauzes and non-woven dressing may be available sterile or non-sterile in bulk and with or without an adhesive border. Exemplary gauze dressings and woven dressings may be used for cleansing, packing and covering a variety of treatment sites.
11) Hydrocolloids: suitable hydrocolloid dressings may include, for example, wafers, powders or pastes composed of gelatin, pectin or carboxymethylcellulose. In certain embodiment, wafers are self-adhering and available with or without an adhesive border and in a wide variety of shapes and sizes. Exemplary hydrocolloids are useful on areas that require contouring. In certain embodiments, powders and pastes hydrocolloids may use used in combination with a secondary dressing.
12) Hydrogels (Amorphous): suitable amorphous hydrogel dressings may include, for example, formulations of water, polymers and other ingredients with no shape, designed to donate moisture and to maintain a moist healing environments and or to rehydrate the treatment site. In certain embodiment, hydrogels may be used in combination with a secondary dressing cover.
13) Hydrogels: Impregnated Dressings: suitable impregnated hydrogel dressings may include, for example, gauzes and non-woven sponges, ropes and strips saturated with an amorphous hydrogel. Amorphous hydrogels may include for example, formulations of water, polymers and other ingredients with no shape, designed to donate moisture to a dry treatment site and to maintain a moist healing environment.
14) Hydrogel Sheets: suitable hydrogel sheets may include for example, three-dimensional networks of cross-linked hydrophilic polymers that are insoluble in water and interact with aqueous solutions by swelling. Exemplary hydrogels are highly conformable and permeable and can absorb varying amounts of drainage, depending on their composition. In certain embodiment, the hydrogel is non-adhesive against the treatment site or treated for easy removal.
15) Impregnated Dressings: suitable impregnated dressings may include, for example, gauzes and non-woven sponges, ropes and strips saturated with a solution, an emulsion, oil, gel or some other pharmaceutically active compound or carrier agent, including for example, saline, oil, zinc salts, petrolatum, xeroform and scarlet red as well as the compounds described herein.
16) Silicone Gel Sheets: suitable silicone gel sheet dressings may include, for example, soft covers composed of cross-linked polymers reinforced with or bonded to mesh or fabric.
17) Solutions: suitable liquid dressings may include, for example, mixtures of multiprotein material and other elements found in the extracellular matrix. In certain embodiment, exemplary solutions may be applied to the treatment site after debridement and cleansing and then covered with an absorbent dressing or a nonadherent pad.
18) Transparent Films: suitable transparent film dressings may include polymer membranes of varying thickness coated on one side with an adhesive. In certain embodiments, transparent films are impermeable to liquid, water and bacteria but permeable to moisture vapor and atmospheric gases. In certain embodiments, the transparency allows visualization of the treatment site.
19) Fillers: suitable filler dressings may include, for example, beads, creams, foams, gels, ointments, pads, pastes, pillows, powders, strands or other formulations. In certain embodiment, fillers are non-adherent and may include a time-released antimicrobial. Exemplary fillers may be useful to maintain a moist environment, manage exudate, and for treatment of for example, partial- and full- thickness wounds, infected wounds, draining wounds and deep wounds that require packing.

### Compositions

The invention includes compositions comprising (a) an effective amount of an anti-connexin 43 agent and (b) a pharmaceutically acceptable carrier or diluent. In one aspect the invention provides a pharmaceutical composition comprising (a) one or more connexin antisense polynucleotides and (b) a pharmaceutically acceptable carrier or diluent. One connexin antisense polynucleotide is an connexin 43 antisense polynucleotide. Additional connexin antisense polynucleotides include connexin 26, 31.1, 32, 36, 40 and 45 antisense polynucleotides. In one embodiment, the connexin antisense polynucleotide comprises the oligodeoxynucleotide set forth in SEQ.ID.NO:1. In one embodiment, the connexin antisense polynucleotide comprises the oligodeoxynucleotide set forth in SEQ.ID.NO:2. In one embodiment, the connexin antisense polynucleotide comprises the oligodeoxynucleotide set forth in SEQ.ID.NO:3. In one embodiment, the connexin antisense polynucleotide comprises the oligodeoxynucleotide set forth in SEQ.ID.NO:4. In one embodiment, the connexin antisense polynucleotide comprises the oligodeoxynucleotide set forth in SEQ.ID.NO:5. In one embodiment, the connexin antisense polynucleotide comprises the oligodeoxynucleotide set forth in SEQ.ID.NO:6. In one embodiment, the connexin antisense polynucleotide comprises the oligodeoxynucleotide set forth in SEQ.ID.NO:7. In one embodiment, the connexin antisense polynucleotide comprises the oligodeoxynucleotide set forth in SEQ.ID.NO:8. In one embodiment, the connexin antisense polynucleotide comprises the oligodeoxynucleotide set forth in SEQ.ID.NO:9. In one embodiment, the connexin antisense polynucleotide comprises the oligodeoxynucleotide set forth in SEQ.ID.NO:10. In one embodiment, the connexin antisense polynucleotide comprises the oligodeoxynucleotide set forth in SEQ.ID.NO:11. In one embodiment, the connexin antisense polynucleotide comprises the oligodeoxynucleotide set forth in SEQ.ID.NO:12.

Described herein is a pharmaceutical composition comprising (a) one or more polynucleotide homologues and (b) a pharmaceutically acceptable carrier or diluent. In one embodiment the polynucleotide homologue is homologous to the oligodeoxynucleotide set forth in SEQ.ID.NO:1. Preferably, at least about 80%, at least about 90 %, at least about 95%, at least about 97%, or at least about 99% homologous to the oligodeoxynucleotide set forth in SEQ.ID.NO:1. In one embodiment the polynucleotide homologue is homologous to the oligodeoxynucleotide set forth in SEQ.ID.NO:2. Preferably at least about 80%, at least about 90 %, at least about 95%, at least about 97%, or at least about 99% homologous to the oligodeoxynucleotide set forth in SEQ.ID.NO:2. In one embodiment the polynucleotide homologue is homologous to the oligodeoxynucleotide set forth in SEQ.ID.NO:3. Preferably, at least about 80%, at least about 90 %, at least about 95%, at least about 97%, or at least about 99% homologous to the oligodeoxynucleotide set forth in SEQ.ID.NO:3. In one embodiment the polynucleotide homologue is homologous to the oligodeoxynucleotide set forth in SEQ.ID.NO:4. Preferably, at least about 80%, at least about 90 %, at least about 95%, at least about 97%, or at least about 99% homologous to the oligodeoxynucleotide set forth in SEQ.ID.NO:4. In one embodiment the polynucleotide homologue is homologous to the oligodeoxynucleotide set forth in SEQ.ID.NO:5. Preferably, at least about 80%, at least about 90 %, at least about 95%, at least about 97%, or at least about 99% to the oligodeoxynucleotide set forth in SEQ.ID.NO:5. In one embodiment the polynucleotide homologue is homologous to the oligodeoxynucleotide set forth in SEQ.ID.NO:6. Preferably, at least about 80%, at least about 90 %, at least about 95%, at least about 97%, or at least about 99% homologous to the oligodeoxynucleotide set forth in SEQ.ID.NO:6. In one embodiment the polynucleotide homologue is homologous to the oligodeoxynucleotide set forth in SEQ.ID.NO:7. Preferably, at least about 80%, at least about 90 %, at least about 95%, at least about 97%, or at least about 99% homologous to the oligodeoxynucleotide set forth in SEQ.ID.NO:7. In one embodiment the polynucleotide homologue is homologous to the oligodeoxynucleotide set forth in SEQ.ID.NO:8. Preferably, at least about 80%, at least about 90 %, at least about 95%, at least about 97%, or at least about 99% homologous to the oligodeoxynucleotide set forth in SEQ.ID.NO:8. In one embodiment the polynucleotide homologue is homologous to the oligodeoxynucleotide set forth in SEQ.ID.NO:9. Preferably, at least about 80%, at least about 90 %, at least about 95%, at least about 97%, or at least about 99% homologous to the oligodeoxynucleotide set forth in SEQ.ID.NO:9. In one embodiment the polynucleotide homologue is homologous to the oligodeoxynucleotide set forth in SEQ.ID.NO:10. Preferably, at least about 80%, at least about 90 %, at least about 95%, at least about 97%, or at least about 99% homologous to the oligodeoxynucleotide set forth in SEQ.ID.NO:10. In one embodiment the polynucleotide homologue is homologous to the oligodeoxynucleotide set forth in SEQ.ID.NO:11. Preferably, at least about 80%, at least about 90 %, at least about 95%, at least about 97%, or at least about 99% homologous to the oligodeoxynucleotide set forth in SEQ.ID.NO: 11. In one embodiment the polynucleotide homologue is homologous to the oligodeoxynucleotide set forth in SEQ.ID.NO:12. Preferably, at least about 80%, at least about 90 %, at least about 95%, at least about 97%, or at least about 99% homologous to the oligodeoxynucleotide set forth in SEQ.ID.NO:12.

Described herein is a pharmaceutical composition comprising (a) one or more binding proteins and (b) a pharmaceutically acceptable carrier or diluent. In one embodiment the binding protein is an antibody. In a preferred embodiment, the binding protein is a monoclonal antibody, polyclonal antibody, antibody fragment, single chain antibodies, single chain Fvs, or single chain binding molecule. In one embodiment the binding protein, for example an antibody, specifically binds to a connexin polypeptide, preferably a connexin 26, connexin 31.1, connexin 32, connexin 36, connexin 40 or connexin 45 polypeptide or any part thereof. In one embodiment the binding protein, for example an antibody, specifically binds to a connexin 43 polypeptide or any part thereof. In one embodiment the binding protein specifically binds to a connexin polypeptide and has an affinity of greater than or equal to about 10⁴ M⁻¹, greater than or equal to about 10⁶ M⁻¹, greater than or equal to about 10⁷ M⁻¹, or greater than or equal to about 10⁸ M⁻¹. Affinities of even greater than about 10⁸ M⁻¹ are suitable, such as affinities equal to or greater than about 10⁹ M⁻¹, about 10¹⁰ M⁻¹, about 10¹¹ M⁻¹, and about 10¹² M⁻¹.

In another embodiment, the pharmaceutical composition comprises (a) one or more binding proteins and (b) a pharmaceutically acceptable carrier or diluent, where in the binding protein is a peptide inhibitor. In one embodiment, the peptide inhibitor comprises an amino acid sequence corresponding to a transmembrane region of connexin 26, connexin 31.1, connexin 32, connexin 36, connexin 40 or connexin 45. In a preferred embodiment, the peptide inhibitor comprises an amino acid sequence corresponding to a transmembrane region of connexin 43. In another embodiment, the peptide inhibitor comprises from about 5-20 contiguous amino acids set forth in SEQ.ID.NO:13. In another embodiment, the peptide inhibitor comprises from about 5-20 contiguous amino acids set forth in SEQ.ID.NO:14. In another embodiment, the peptide inhibitor comprises an amino acid sequence corresponding to the regions at positions 37-76 and 178-208 of SEQ.ID.NO:14. In another embodiment, the peptide inhibitor comprises from about 5-20 contiguous amino acids set forth in SEQ.ID.NO:15, SEQ.ID.NO:16, SEQ.ID.NO:17, SEQ.ID.NO:18, SEQ.ID.NO:19, SEQ.ID.NO:20, SEQ.ID.NO:21, SEQ.ID.NO:22, SEQ.ID.NO:23, SEQ.ID.NO:24, SEQ.ID.NO:25, SEQ.ID.NO:26, SEQ.ID.NO:27, SEQ.ID.NO:28, SEQ.ID.NO:29, SEQ.ID.NO:30, SEQ.ID.NO:31, SEQ.ID.NO:32, SEQ.ID.NO:33, SEQ.ID.NO:34, SEQ.ID.NO:35, SEQ.ID.NO:36, SEQ.ID.NO:37, or SEQ.ID.NO:38. In another embodiment, the peptide inhibitor comprises from about 8 to about 11 contiguous amino acids set forth in SEQ.ID.NOs: 39-66. In another embodiment, the peptide inhibitor comprises from about 8 to about 15 contiguous amino acids set forth in SEQ.ID.NOs: 18, 19, or 67-80. In yet another embodiment, the peptide inhibitor has one or more conservative amino acid changes.

In another aspect of the invention the composition comprises (a) an effective amount of an anti-connexin 43 agent, (b) one or more therapeutic agents, and (c) a pharmaceutically acceptable carrier or diluent. Therapeutic agents include for example, for example, anti-infectives, anaesthetics, analgesics, antibiotics, narcotics, and steroidal and non-steroidal anti-inflammatory agents. In certain embodiments, one, two three, four, five or six therapeutic agents may be used in combination.

In another aspect of the invention the composition comprises (a) an effective amount of an anti-connexin 43 agent, (b) one or more agents useful for wound healing, and (c) a pharmaceutically acceptable carrier or diluent. Agents useful for wound healing may include, for example, a wound healing associated growth factor, and/or a cytokine In certain embodiments, one, two three, four, five or six agents useful for wound healing may be used in combination.

In another aspect of the invention the composition comprises (a) an effective amount of an anti-connexin 43 agent, (b) one or more anti-microtubule agents, and (c) a pharmaceutically acceptable carrier or diluent. Exemplary anti-microtubule agents include, for example, diterpenoids, vinca alkaloids, and platinum coordination complexes. In certain embodiments, one, two three, four, five or six und anti-microtubule agents may be used in combination.

Any of the methods of treating a subject having or suspected of having or predisposed to, or at risk for, a disease, disorder, and/or condition, referenced or described herein may utilize the administration of any of the doses, dosage forms, formulations, and/or compositions herein described.

### Treatment

The application describes methods for treating and/or preventing, in whole or in part, various diseases, disorders and conditions, including, for example, methods of treating a subject having or suspected of having or predisposed to, or at risk for various orthopedic-related diseases, disorders, or conditions and diseases, disorders or conditions characterized in whole or in part by abnormal tissue formation inside and/or around a joint, comprising administering a composition comprising an anti-connexin agent and a pharmaceutically acceptable carrier or diluent. Such compounds may be administered in amounts, for example, that are effective to (1) improve recovery time in a patient after orthopedic surgery, (2) decrease pain in subject after orthopedic surgery (3) improve overall recovery results in a subject after orthopedic surgery, (4) prevent and/or decrease abnormal tissue formation inside and/or around a joint, and/or (5) prevent and/or decrease vascular damage. Such compounds may be administered in amounts, for example, that are effective to prevent or decrease joint contracture.

The application also discloses methods of treating and/or preventing, in whole or in part, various diseases, disorders and conditions associated with orthopedic surgical procedures, including, for example, methods of promoting would-healing following said orthopedic surgical procedures comprising administering a composition comprising an anti-connexin agent and a pharmaceutically acceptable carrier or diluent. The invention also includes methods of treating and/or preventing, in whole or in part, various diseases, disorders and conditions associated with orthopedic surgical procedures, including, for example, methods of preventing or decreasing joint contracture following said orthopedic surgical procedures comprising administering a composition comprising an anti-connexin agent and a pharmaceutically acceptable carrier or diluent. The invention also includes methods of treating and/or preventing, in whole or in part, various diseases, disorders and conditions associated with orthopedic surgical procedures, including, for example, methods of treating diseases, disorders, or conditions and diseases, disorders or conditions characterized in whole or in part by tissue formation inside and/or around a joint associated with said orthopedic surgical procedures comprising administering a composition comprising an anti-connexin agent and a pharmaceutically acceptable carrier or diluent. Such compounds may be administered in amounts, for example, that are effective to (1) improve recovery time in a patient after orthopedic surgery, (2) decrease pain in subject after orthopedic surgery (3) improve overall recovery results in a subject after orthopedic surgery, (4) prevent and/or decrease abnormal tissue formation inside and/or around a joint and/or (5) prevent and/or decrease vascular damage. Such compounds may be administered in amounts, for example, that are effective to prevent or decrease joint contracture. Such compounds may be administered in amounts, for example, that are effective to downregulate expression of connexin proteins at and immediately adjacent the surgical site.

The application also discloses methods of treating a subject having orthopedic surgery, by administering a composition comprising an anti-connexin agent, wherein recovery time in said subject is improved. In one embodiment, the recovery time in said patient is shorter than the recovery time when untreated. The invention also includes methods of treating a subject having orthopedic surgery, by administering a composition comprising an anti-connexin agent, wherein pain is decreased in said subject. The invention also includes methods of treating a subject having orthopedic surgery, by administering a composition comprising an anti-connexin agent, wherein the overall recovery results are improved. In one embodiment, improved recovery result are demonstrated by increased post-operative mobility. The invention also includes methods of treating and/or preventing, in whole or in part, post-orthopedic-surgical joint contracture by administering a composition comprising an anti-connexin agent. Such compositions may be administered alone or in combination with other therapeutic agents into or around the affected site, including for example, orthopedic surgical wound sites including but not limited to peritendinal, soft tissue injection, intra-articular and peri-articular joints. Such compositions may be administered as mono-therapy, concurrent or intra-operatively in combination with corrective orthopedic procedures, or post-procedurally. Repeat applications are included within the invention.

The application discloses methods for treating and/or preventing, in whole or in part, various diseases, disorders and conditions, including, for example, orthopedic diseases, disorders, and conditions including, for example, anterior cruciate ligament (acl) injury; arthritis of the shoulder; articular cartilage injury of the knee; bowlegs; broken back; broken hip (pelvis); broken leg; broken neck; bunions; bursitis; carpal tunnel syndrome; chronic low back pain; clubfoot; curvature of the spine (scoliosis); diabetic foot; dislocated elbow; dislocated hip; dupuytren's contracture; flatfoot; foot deformity; forearm fractures in children; hemophiliac arthritis; herniated disc (slipped disc); hip labral tear; hip arthritis; infectious arthritis; inflammatory hip conditions; intoeing; knee arthritis; knock knee; meniscal tear; osteoarthritis; osteonecrosis; osteoporosis; rheumatoid arthritis; rotator cuff injuries; scoliosis; shoulder arthritis; shoulder instability; shoulder pain; slipped disc (herniated disc); spinal stenosis/degenerative spondylolisthesis; spondylolisthesis; sports injuries to foot; sprained ankle; tendonitis; thumb arthritis; and trigger finger.

Any one of the methods of treatment described herein may further comprise administration of second composition having one or more drugs effective in (1) preventing and/or decreasing pain, (2) improving post-operative recovery times, (3) improving overall recovery outcome in post-orthopedic-surgical subject, (4) preventing and/or decreasing joint contracture and/or (5) prevent and/or decrease vascular damage. The second composition may comprise an anti-connexin agent. The second composition may comprise one or more therapeutic agents. The second composition may comprise one or more one or more anti-microtubule agents. The second composition may be administered before, after and/or simultaneously with the first composition. The second composition may be administered before and after the first composition. The second composition may be administered simultaneously with the first composition.

### Kits and Articles of Manufacture

In one aspect, the application discloses a kit for treating orthopedic diseases, disorders and conditions, promoting orthopedic related surgical wound healing, improving recovery time, and/or decreasing post-operative pain, and/or improving overall recovery results in a post-operative subject and/or prevent and/or decrease vascular damage, preventing and/or treating abnormal tissue formation inside and/or around a joint, and/or preventing, decreasing or reversing of joint contracture.

The kit may include one or more compositions described herein. For example, the kit may include a composition comprising an effective amount of an anti-connexin agent. In one embodiment, the kit comprises a composition that comprises an effective amount of one or more connexin antisense polynucleotides. The kit may comprise a composition that comprises an effective amount of one or more polynucleotide homologues. In one embodiment, the kit comprises a composition that includes an effective amount of one or more peptide or polypeptide anti-connexin agents.

In another aspect, the application describes an article of manufacture comprising a vessel containing an effective amount of an anti-connexin agent, e.g., connexin antisense polynucleotides, and instructions for use, including use for the treatment of a subject having or suspected of having or predisposed to any of the diseases, disorders and/or conditions described or referenced herein, including orthopedic diseases, disorders and/or conditions, wherein the packaging material has a label that indicates that the dosage form can be used for a subject.

In certain other aspect, the application describes an article of manufacture comprising a vessel containing an effective amount of an anti-connexin agent, *e.g.,* a connexin antisense polynucleotide, together with instructions for use for the treatment of a subject undergoing an orthopedic surgical procedure

A better understanding of the invention will be gained by reference to the following experimental section. The following experiments are illustrative and are not intended to limit the invention or the claims in any way.

### EXAMPLES

### EXAMPLE 1

### Maximum Tolerated Dose Determination by Intra-articular Injection

Male Hartley guinea pigs, at least 6 weeks old, are anaesthetized using 5% isoflurane in an enclosed chamber. The animals are weighed and then transferred to the surgical table where anesthesia was maintained by nose cone with 2% isoflurane. The knee area on both legs are shaved and knee width at the head of the femur is measured on both knees. The skin on the right knee is sterilized. A 25G needle is introduced into the synovial cavity using a medial approach and 0.1 ml of the test formulations is injected. Seven days after the injection, the animals are sacrificed by cardiac injection of 0.7 ml Euthanyl under deep anesthesia (5% isoflurane).

Assessment of tolerability: Knee function is assessed before sacrifice by recording changes in walking behavior and signs of tenderness. The animal is weighed immediately after sacrifice. The width of both knees at the head of the femur is then measured with calipers. The knee joint is dissected open by transecting the quadriceps tendon, cutting through the lateral and medial articular capsule and flipping the patella over the tibia. Knee inflammation is assessed by recording signs of swelling, vascularization, fluid accumulation and change in color in subcutaneous tissue as well as inner joint structures.

### EXAMPLE 2

### Spinal Surgery Model

The rabbit laminectomy spinal adhesion model described herein is used to investigate prevention of spinal scarring and abnormal tissue formation (e.g. adhesion) by local administration of an anti-connexin agent. Five to six animals will be included in each experimental group to allow for meaningful statistical analysis. Formulations with various concentrations of anti-connexin agent are tested against control animals to assess inhibition of scarring and adhesion formation.

Rabbits are anesthetized with an IM injection of ketamine/zylazine. An endotracheal tube is inserted for maintenance of anesthesia with halothane. The animal is placed prone on the operating table on top of a heating pad and the skin over the lower half of the back is shaved and prepared for sterile surgery. A longitudinal midline skin incision is made from L-1 to L-5 and down the lumbosacral fascia. The fascia is incised to expose the tips of the spinous processes. The paraspinous muscles are dissected and retracted from the spinous process and lamina of L-4. A laminectomy is performed at L-4 by removal of the spinal process with careful bilateral excision of the laminae, thus creating a small 5x10 mm laminectomy defect. Hemostasis is obtained with Gelfoam. The test formulations are applied to the injury site and the wound is closed in layers with Vicryl sutures. The animals are placed in an incubator until recovery from anesthesia and then returned to their cage.

Two weeks after surgery, the animals are anesthetized using procedures similar to those described above. The animals are euthanized with Euthanyl. After a skin incision, the laminectomy site is analyzed by dissection and the amount of scarring and adhesion is scored using scoring systems published in the scientific literature for this type of injury.

### EXAMPLE 3

### Tendon Surgery Model

This model is used to investigate whether scarring and adhesion of tendons can be prevented by localized administration of anti-connexin agents. Extended release formulations are loaded with drugs and implanted around injured tendons in rabbits.

Rabbits are anesthetized and the skin over the right hind limb is shaved and prepared for sterile surgery. Sterile surgery is performed aided by an operating microscope. A longitudinal midline skin incision is made on the volvar aspect of the proximal phalange in digits 2 and 4. The synovial sheath of the tendons is carefully exposed and incised transversally to access the flexor digitorum profundus distal to the flexor digitorum superficialis bifurcation. Tendon injury is performed by gently lifting the flexor digitorum profundus with curved forceps and incising transversally through half of its substance. The test formulations are applied around the tendons in the sheath of one of the two digits randomly selected. The other digit is left untreated and is used as a control. The sheath is then repaired with 6-0 nylon suture. An immobilizing 6-0 nylon suture is inserted through the transverse metacarpal ligament into the tendon/sheath complex to immobilize the tendon and the sheath as a single unit. The wound is closed with 4-0 interrupted sutures. A bandage is applied around the hind paw to further augment immobilization of the digits and ensure comfort and ambulation of the animals. The animals are recovered and returned to their cage.

Three weeks after surgery, the animals are anesthetized. After a skin incision, the tissue plane around the synovial sheath is dissected and the tendon-sheath complex harvested en block and transferred in 10% phosphate buffered formaldehyde for histopathology analysis. The animals are then euthanized. After paraffin embedding, serial 5 µm thin cross-sections are cut every 2 mm through the sheath and tendon complex. Sections are stained with H&E and Movat's stains to evaluate adhesion growth. Each slide is digitized using a computer connected to a digital microscope camera (Nikon Micropublisher cooled camera). Morphometry analysis is then performed using image analysis software (ImagePro). Thickness and area of adhesion defined as the substance obliterating the synovial space are measured and compared between formulation-treated and control animals.

### EXAMPLE 4

Anti-corinexin agent is conveniently formulated in a form suitable for administration according to the methods of the present invention.

Suitable formulations include a mixture of the following formulating agents. The amount of the individual aniti-connexin agent or agents and formulating agents will depend on the particular use intended.

| |
|---|
| ASO in PBS |
| Polyquarternium 10 |
| HEC/HPMC/CMC |
| Na Hyaluronate |
| Tween 20 |
| Poloxamer 188 |
| Pluronic 87 NF |
| SLES |
| Poly L-lysine/Polyethylene Imine |
| Banzalkonium chloride |
| Methyl paraben |
| Proplparaben |
| Propylene Glycol |
| 10mM Phosphate Buffer |

### EXAMPLE 5

Formulations for use according to methods of the present invention are prepared by mixing the compounds in the proportions noted below. In one preferred embodiment, the anti-connexin agent is an anti-connexin polynucleotide. In other embodiments, the anti-connexin polynucleotide is an anti-sense oligonucleotide, for example, an antisense oligonucleotide of SEQ. ID. NO. 1.

### Formulation A

Made up of the following materials (% w/w) - Anti-connexin agent in phosphate-buffered saline (0.47%); Methylparaben (0.17%); Propylparaben (0.03%); Propylene Glycol (1.5%); HPMC (1.5%); and 10 mM Phosphate Buffer (96.33%). Formulation is a clear gel with pH ∼6.74 and osmolality of 244.

### Formulation B

Made up of the following materials (% w/w) - Anti-connexin agent in phosphate-buffered saline (0.47%); Methylparaben (0.17%); Propylparaben (0.03%); Propylene Glycol (1.5%); HPMC (1.5%); 0.5% BAC (0.1%); and 10 mM Phosphate Buffer (96.23%). Formulation is a clear gel with pH ∼6.65 and osmolality of 230.

### Formulation C

Made up of the following materials (% w/w) - Anti-connexin agent in phosphate-buffered saline (0.47%); Methylparaben (0.17%); Propylparaben (0.03%); Propylene Glycol (1.5%); HPMC (1.5%); Polyquaternium 10 (0.5%); Poloxamer 188 (0.1%); and 10 mM Phosphate Buffer (95.73%). Formulation is a slightly hazy gel with pH ∼6.59 and osmolality of 233.

### Formulation D

Made up of the following materials (% w/w) - Anti-connexin agent in phosphate-buffered saline (0.47%); Methylparaben (0.17%); Propylparaben (0.03%); Propylene Glycol (1.5%); HPMC (1.5%); SLES (0.5%); and 10 mM Phosphate Buffer (95.83%). Formulation is a clear gel with pH ∼6.8 and osmolality of 246.

### Formulation E

Made up of the following materials (% w/w) - Anti-connexin agent in phosphate-buffered saline (0.47%); Methylparaben (0.17%); Propylparaben (0.03%); Propylene Glycol (1.5%); HPMC (1.5%); Poloxamer 188 (0.1%); 25K Polyethylene Imine (0.075%); and 10 mM Phosphate Buffer (96.155%). Formulation is a hazy gel with pH ∼7.8 and osmolality of 249.

### Formulation F

Made up of the following materials (% w/w) - Anti-connexin agent in phosphate-buffered saline (0.47%); Methylparaben (0.17%); Propylparaben (0.03%); Propylene Glycol (1.5%); HPMC (1.5%); Sodium Hyaluronate (0.1%); and 10 mM Phosphate Buffer (96.23%). Formulation is a clear gel with pH ∼6.88 and osmolality of 289.

### Formulation G

Made up of the following materials (% w/w) - Anti-connexin agent in phosphate-buffered saline (0.47%); Methylparaben (0.17%); Propylparaben (0.03%); Propylene Glycol (1.5%); Sodium Hyaluronate (1.0%); and 10 mM Phosphate Buffer (96.83%). Formulation is a clear gel with pH ∼6.81 and osmolality of 248.

## Claims

1. A composition comprising an anti-connexin 43 agent for use in a method of improving recovery time, reducing pain, decreasing joint contracture and/or improving mobility in a subject undergoing an orthopedic surgery comprising administration of said composition into or around the surgical site within said subject before, at the time of, or after said orthopedic procedure.

2. A composition for use according to claim 1, wherein said method is a method of improving recovery time reducing pain and/or improving mobility.

3. A composition for use according to claim 1, wherein said method is a method of decreasing pain in a post-orthopaedic-surgical subject comprising administration of said composition during or at the conclusion of said surgery.

4. A composition for use according to claim 1, wherein said method is a method of treating and/or preventing, in whole or in part, post-orthopedic-surgical joint contracture in a subject by administering said composition to said subject during or after said orthopedic surgery.

5. The composition for use according to claim 4, wherein said subject is further administered a second composition comprising an anti-connexin agent.

6. A composition for use according to claim 4, wherein said method is a method of preventing or decreasing pain or excess scarring inside and/or around a joint in said subject.

7. A composition comprising an effective amount of an anti-connexin 43 agent for use in a method of reducing pain and/or improving mobility in a subject having or suspected of having an orthopedic disease, disorder, or condition **characterized in** whole or in part by pain and/or excess scarring inside and/or around a joint.

8. A composition for use according to any one of claims 1 to 4 and 7, wherein said anti-connexin 43 agent is:
(a) a connexin 43 polynucleotide;
(b) an anti-connexin 43 antisense polynucleotide;
(c) an anti-connexin 43 polynucleotide that decreases connexin 43 protein expression;
(d) an siRNA or and RNAi oligonucleotide;
(e) a peptide;
(f) a peptide that is a peptidomimetic;
(g) an anti-connexin 43 antibody or antigen binding fragment thereof; or
(h) an antibody, F(v) fragment, Fab fragment, Fab' fragment, or F(ab')2 fragment.

9. A composition for use according to any one of claims 1 to 4 and 7, wherein the anti-connexin 43 agent:
(a) inhibits or blocks intercellular communication; or
(b) inhibits or blocks connexin 43 hemichannel opening.

10. A composition for use according to any one of claims 1 to 4 and 7, wherein said method further comprises:
(a) administration of second composition comprising one or more therapeutic agents;
(b) administration of second composition comprising one or more agents useful for wound healing; or
(c) administration of second composition comprising one or more anti-microtubule agents.

11. A composition for use according to claim 10 or claim 5, wherein the second composition is administered:
(a) before, after or simultaneously with the first composition; or
(b) before and after the first composition.

12. A composition for use according to any one of claims 1 to 4 and 7, wherein abnormal tissue formation inside and/or around a joint is prevented or decreased.

13. A compound for use according to claim 8 wherein the anti-connexin 43 compound is a chemically modified antisense polynucleotide.

14. A composition for use according to claim 8, wherein said anti-connexin 43 agent is a connexin 43 polynucleotide comprising SEQ ID NO:1, SEQ ID NO:2, or SEQ ID NO:3.

15. A compound for use according to claim 8 wherein:
(a) said peptide comprises an amino acid sequence corresponding to a portion of an extracellular loop region or a portion of a transmembrane region of connexin 43, wherein the peptide comprises SEQ ID NO: 18, SEQ ID NO: 19, SEQ ID NO: 35, or a portion of SEQ ID NO: 67 or SEQ ID NO: 62; or
(b) said peptide has an amino acid sequence that comprises 5 to 20, 8 to 15, or 11 to 13 contiguous amino acids of an extracellular loop region or transmembrane region of SEQ ID NO: 14.

16. A compound for use according to claim 15 wherein said peptide comprises the amino acid sequence shown in SEQ ID NO: 35.

17. A compound for use according to claim 16 wherein said peptide comprises the amino acid sequence shown in SEQ ID NO: 18.

## Patentansprüche

1. Zusammensetzung, umfassend ein Anti-Connexin-43-Agens zur Verwendung bei einem Verfahren zur Verbesserung der Genesungszeit, Reduzierung von Schmerzen, Verminderung von Gelenkkontraktur und/oder Verbesserung der Beweglichkeit bei einem Patienten, der orthopädisch-chirurgisch behandelt wird, umfassend die Verabreichung der Zusammensetzung an die Stelle des chirurgischen Eingriffs oder um diese herum bei dem Patienten, und zwar vor dem, während des oder nach dem orthopädischen Eingriff(s).

2. Zusammensetzung zur Verwendung nach Anspruch 1, wobei das Verfahren ein Verfahren zur Verbesserung der Genesungszeit, Reduzierung von Schmerzen und/oder Verbesserung der Beweglichkeit ist.

3. Zusammensetzung zur Verwendung nach Anspruch 1, wobei das Verfahren ein Verfahren zur Verringerung von Schmerzen bei einem Patienten nach orthopädisch-chirurgischer Behandlung ist, umfassend die Verabreichung der Zusammensetzung während oder bei Beendigung des chirurgischen Eingriffs.

4. Zusammensetzung zur Verwendung nach Anspruch 1, wobei das Verfahren ein Verfahren zur Behandlung und/oder Prävention ist, und zwar ganz oder teilweise bezüglich der Gelenkkontraktur nach orthopädisch-chirurgischer Behandlung bei einem Patienten, und zwar durch Verabreichung der Zusammensetzung an den Patienten während des oder nach dem orthopädischen Eingriff(s).

5. Zusammensetzung zur Verwendung nach Anspruch 4, wobei dem Patienten des Weiteren eine zweite Zusammensetzung, umfassend ein Anti-Connexin-Agens, verabreicht wird.

6. Zusammensetzung zur Verwendung nach Anspruch 4, wobei das Verfahren ein Verfahren zur Prävention oder Verringerung von Schmerzen oder übermäßiger Vernarbung innerhalb eines Gelenks und/oder um ein Gelenk herum bei dem Patienten ist.

7. Zusammensetzung, umfassend eine wirksame Menge eines Anti-Connexin-43-Agens zur Verwendung bei einem Verfahren zur Reduzierung von Schmerzen und/oder Verbesserung der Beweglichkeit bei einem Patienten, der an einer orthopädischen Krankheit, Erkrankung oder einem orthopädischen Zustand leidet oder bei dem ein entsprechender Verdacht besteht, gekennzeichnet, ganz oder teilweise, durch Schmerzen und/oder übermäßige Vernarbung innerhalb eines Gelenks und/oder um ein Gelenk herum.

8. Zusammensetzung zur Verwendung nach einem der Ansprüche 1 bis 4 und 7, wobei das Anti-Connexin-43-Agens ist:
(a) ein Connexin-43-Polynucleotid;
(b) ein Anti-Connexin-43-Antisense-Polynucleotid;
(c) ein Anti-Connexin-43-Polynucleotid, das die Connexin-43-Proteinexpression verringert;
(d) ein siRNA- oder/und RNAi-Oligonucleotid;
(e) ein Peptid;
(f) ein Peptid, das ein Peptidmimetikum ist;
(g) ein Anti-Connexin-43-Antikörper oder ein Antigen-Bindungsfragment davon; oder
(h) ein Antikörper, F(v)-Fragment, Fab-Fragment, Fab'-Fragment oder F(ab')2-Fragment.

9. Zusammensetzung zur Verwendung nach einem der Ansprüche 1 bis 4 und 7, wobei das Anti-Connexin-43-Agens:
(a) die intrazelluläre Kommunikation hemmt oder blockiert; oder
(b) die Öffnung von Connexin-43-Hemikanälen hemmt oder blockiert.

10. Zusammensetzung zur Verwendung nach einem der Ansprüche 1 bis 4 und 7, wobei das Verfahren des Weiteren umfasst:
(a) die Verabreichung einer zweiten Zusammensetzung, umfassend ein oder mehr therapeutische Mittel;
(b) die Verabreichung einer zweiten Zusammensetzung, umfassend ein oder mehr Mittel, die für die Wundheilung nützlich sind; oder
(c) die Verabreichung einer zweiten Zusammensetzung, umfassend ein oder mehr Anti-Mikrotubulus-Agenzien.

11. Zusammensetzung zur Verwendung nach Anspruch 10 oder Anspruch 5, wobei die zweite Zusammensetzung verabreicht wird:
(a) vor, nach oder gleichzeitig mit der ersten Zusammensetzung; oder
(b) vor und nach der ersten Zusammensetzung.

12. Zusammensetzung zur Verwendung nach einem der Ansprüche 1 bis 4 und 7, wobei abnormale Gewebebildung innerhalb eines Gelenks und/oder um ein Gelenk herum verhindert oder verringert wird.

13. Verbindung zur Verwendung nach Anspruch 8, wobei die Anti-Connexin-43-Verbindung ein chemisch modifiziertes Antisense-Polynucleotid ist.

14. Zusammensetzung zur Verwendung nach Anspruch 8, wobei das Anti-Connexin-43-Agens ein Connexin-43-Polynucleotid ist, das SEQ ID NO:1, SEQ ID NO:2 oder SEQ ID NO:3 umfasst.

15. Verbindung zur Verwendung nach Anspruch 8, wobei:
(a) das Peptid umfasst: eine Aminosäuresequenz entsprechend einem Teil einer extrazellulären Schleifenregion oder einem Teil einer Transmembran-Region von Connexin-43, wobei das Peptid umfasst: SEQ ID NO:18, SEQ ID NO:19, SEQ ID NO:35 oder einen Teil von SEQ ID NO:67 oder SEQ ID NO:62; oder
(b) das Peptid eine Aminosäuresequenz aufweist, die 5 bis 20, 8 bis 15 oder 11 bis 13 zusammenhängende Aminosäuren einer extrazellulären Schleifenregion oder Transmembranregion von SEQ ID NO:14 umfasst.

16. Verbindung zur Verwendung nach Anspruch 15, wobei da Peptid die in SEQ ID NO:35 gezeigte Aminosäuresequenz umfasst.

17. Verbindung zur Verwendung nach Anspruch 16, wobei das Peptid die in SEQ ID NO:18 gezeigte Aminosäuresequenz umfasst.

## Revendications

1. Composition comprenant un agent anti-connexine 43 pour utilisation dans un procédé d'amélioration du temps de rétablissement, de réduction de la douleur, de diminution des contractures articulaires et/ou d'amélioration de la mobilité chez un sujet subissant une intervention chirurgicale orthopédique, comprenant l'administration de ladite composition dans le ou autour du site chirurgical chez ledit sujet avant, pendant ou après ladite opération orthopédique.

2. Composition pour utilisation selon la revendication 1, dans laquelle ledit procédé est un procédé d'amélioration du temps de rétablissement, de réduction de la douleur et/ou d'amélioration de la mobilité.

3. Composition pour utilisation selon la revendication 1, dans laquelle ledit procédé est un procédé de diminution de la douleur chez un sujet après intervention chirurgicale orthopédique, comprenant l'administration de ladite composition au cours ou à la fin de ladite intervention chirurgicale.

4. Composition pour utilisation selon la revendication 1, dans laquelle ledit procédé est un procédé de traitement et/ou de prévention, en partie ou en totalité, des contractures articulaires postopératoires en chirurgie orthopédique chez un sujet par administration de ladite composition audit sujet pendant ou après ladite opération orthopédique.

5. Composition pour utilisation selon la revendication 4, dans laquelle on administre encore audit sujet une seconde composition comprenant un agent anti-connexine.

6. Composition pour utilisation selon la revendication 4, dans laquelle ledit procédé est un procédé de prévention ou de diminution de la douleur ou de la cicatrisation excessive à l'intérieur et/ou autour d'une articulation chez ledit sujet.

7. Composition comprenant une quantité efficace d'un agent anti-connexine 43 pour utilisation dans un procédé de réduction de la douleur et/ou d'amélioration de la mobilité chez un sujet ayant ou suspecté d'avoir une maladie, un trouble ou un état orthopédique caractérisé(e) en totalité ou en partie par une douleur et/ou une cicatrisation excessive à l'intérieur et/ou autour d'une articulation.

8. Composition pour utilisation selon l'une quelconque des revendications 1 à 4 et 7, dans laquelle ledit agent anti-connexine 43 est :
(a) un polynucléotide de connexine 43 ;
(b) un polynucléotide antisens anti-connexine 43 ;
(c) un polynucléotide anti-connexine 43 qui diminue l'expression de la protéine connexine 43 ;
(d) un oligonucléotide d'ARNsi et/ou d'ARNi ;
(e) un peptide ;
(f) un peptide qui est un peptidomimétique ;
(g) un anticorps anti-connexine 43 ou un fragment de celui-ci se liant à l'antigène ; ou
(h) un anticorps, fragment F(v), fragment Fab, fragment Fab' ou fragment F(ab')2.

9. Composition pour utilisation selon l'une quelconque des revendications 1 à 4 et 7, dans laquelle l'agent anti-connexine 43 :
(a) inhibe ou bloque la communication intercellulaire ; ou
(b) inhibe ou bloque l'ouverture des hémi-canaux de la connexine 43.

10. Composition pour utilisation selon l'une quelconque des revendications 1 à 4 et 7, dans laquelle ledit procédé comprend en outre :
(a) l'administration d'une seconde composition comprenant un ou plusieurs agents thérapeutiques ;
(b) l'administration d'une seconde composition comprenant un ou plusieurs agents utiles pour la guérison de plaies ; ou
(c) l'administration d'une seconde composition comprenant un ou plusieurs agents anti-microtubules.

11. Composition pour utilisation selon la revendication 10 ou la revendication 5, dans laquelle on administre la seconde composition :
(a) avant, après ou en même temps que la première composition, ou
(b) avant et après la première composition.

12. Composition pour utilisation selon l'une quelconque des revendications 1 à 4 et 7, dans laquelle la formation de tissu anormal à l'intérieur et/ou autour d'une articulation est empêchée ou diminuée.

13. Composé pour utilisation selon la revendication 8, dans lequel le composé anti-connexine 43 est un polynucléotide antisens modifié chimiquement.

14. Composition pour utilisation selon la revendication 8, dans laquelle ledit agent anti-connexine 43 est un polynucléotide de connexine 43 comprenant une séquence présentée dans la Séquence N° 1, la Séquence N° 2 ou la Séquence N° 3.

15. Composé pour utilisation selon la revendication 8, dans lequel :
(a) ledit peptide comprend une séquence d'acides aminés correspondant à une partie d'une région de boucle extracellulaire ou une partie d'une région transmembranaire de connexine 43, le peptide comprenant la séquence présentée dans la Séquence N° 18, la Séquence N° 19, la Séquence N° 35 ou une partie de la Séquence N° 67 ou de la Séquence N° 62 ; ou
(b) ledit peptide a une séquence d'acides aminés qui comprend 5 à 20, 8 à 15 ou 11 à 13 acides aminés contigus d'une région de boucle extracellulaire ou région transmembranaire de la séquence présentée dans la Séquence N° 14.

16. Composé pour utilisation selon la revendication 15, dans lequel ledit peptide comprend la séquence d'acides aminés présentée dans la Séquence N° 35.

17. Composé pour utilisation selon la revendication 16, dans lequel ledit peptide comprend la séquence d'acides aminés présentée dans la Séquence N° 18.
